# EUROPEAN PATENT APPLICATION

(11) **EP 2 671 614 A1**
(43) Date of publication of application: **11.12.2013**
(21) Application number: 13178092.6
(22) Date of filing: 08.08.2008
(51) Int. Cl.: A61P 29/00, A61K 38/17

(54) **Molecular Targets for Treatment of Inflammation**

(30) Priority: 21.12.2007 US 16179 P
(62) Divisional of application: 08782697.0
(71) Applicant: University of Rochester, Rochester, NY 14642 (US)
(72) Inventor: Rahman, Irfan, Rochester, NY New York 14620 (US); Yao, Hongwei, Rochester, NY New York 14620 (US); Rajendrasozhan, Saravanan, Rochester, NY New York 14620 (US)
(74) Representative: Jappy, John William Graham

(57) **Abstract**

Molecular targets and methods for treating inflammatory disorders are described. The expression and/or functionality of molecular targets RelB or SIRT1 is modified in order to treat inflammatory disorders. The expression of RelB or SIRT1 may be increased, or the activity of RelB or SIRT1 may be increased in order to inhibit transcription factors which activate genes involved in inflammation. Inflammatory disorders such as chronic obstructive pulmonary disorder, rheumatoid arthritis, asthma and idiopathic pulmonary fibrosis are indicated.

SEQUENCE LISTING

<110> University of Rochester

<120> Molecular Targets for Treatment of Inflammation

<130> JWJ01647EP1

<160> 10

<170> PatentIn version 3.3

<210> 1
<211> 558
<212> PRT
<213> Homo sapiens

<400> 1

Met Pro Ser Arg Arg Ala Ala Arg Glu Ser Ala Pro Glu Leu Gly Ala
1               5                   10                  15

Leu Gly Ser Ser Asp Leu Ser Ser Leu Ser Leu Thr Val Ser Arg Thr
            20                  25                  30

Thr Asp Glu Leu Glu Ile Ile Asp Glu Tyr Ile Lys Glu Asn Gly Phe
        35                  40                  45

Gly Leu Val Gly Thr Gln Leu Ser Glu Met Pro Arg Leu Val Pro Arg
    50                  55                  60

Gly Pro Ala Ser Leu Ser Ser Val Thr Leu Gly Pro Ala Ala Pro Pro
65                  70                  75                  80

Pro Pro Ala Thr Pro Ser Trp Ser Cys Thr Leu Gly Arg Leu Val Ser
                85                  90                  95

Pro Gly Pro Cys Pro Arg Pro Tyr Leu Val Ile Thr Glu Gln Pro Lys
            100                 105                 110

Gln Arg Gly Met Arg Phe Arg Tyr Glu Cys Glu Gly Arg Ser Ala Gly
        115                 120                 125

Ser Ile Leu Gly Glu Ser Ser Thr Glu Ala Ser Lys Thr Gln Pro Ala
    130                 135                 140

Ile Glu Leu Arg Asp Cys Gly Gly Leu Arg Glu Val Glu Val Thr Ala
145                 150                 155                 160

Cys Leu Val Trp Lys Asp Trp Pro His Arg Val His Pro His Ser Leu
                165                 170                 175

Val Gly Lys Asp Cys Thr Asp Gly Val Cys Arg Val Arg Leu Arg Pro
            180                 185                 190

His Val Ser Pro Arg His Ser Phe Asn Asn Leu Gly Ile Gln Cys Val

        195                 200                 205

Arg Lys Lys Glu Ile Glu Ala Ala Ile Glu Arg Lys Ile Gln Leu Gly
    210                 215                 220

Ile Asp Pro Tyr Asn Ala Gly Ser Leu Lys Asn His Gln Glu Val Asp
225                 230                 235                 240

Met Asn Val Val Arg Ile Cys Phe Gln Ala Ser Tyr Arg Asp Gln Gln
                245                 250                 255

Gly His Leu His Arg Met Asp Pro Ile Leu Ser Glu Pro Val Tyr Asp
            260                 265                 270

Lys Lys Ser Thr Asn Thr Ser Glu Leu Arg Ile Cys Arg Ile Asn Lys
        275                 280                 285

Glu Ser Gly Pro Cys Thr Gly Gly Glu Glu Leu Tyr Leu Leu Cys Asp
    290                 295                 300

Lys Val Gln Lys Glu Asp Ile Ser Val Val Phe Ser Thr Ala Ser Trp
305                 310                 315                 320

Glu Gly Arg Ala Asp Phe Ser Gln Ala Asp Val His Arg Gln Ile Ala
                325                 330                 335

Ile Val Phe Lys Thr Pro Pro Tyr Glu Asp Leu Glu Ile Ser Glu Pro
            340                 345                 350

Val Thr Val Asn Val Phe Leu Gln Arg Leu Thr Asp Gly Val Cys Ser
        355                 360                 365

Glu Pro Leu Pro Phe Thr Tyr Leu Pro Arg Asp His Asp Ser Tyr Gly
    370                 375                 380

Val Asp Lys Lys Arg Lys Arg Gly Leu Pro Asp Val Leu Gly Glu Leu
385                 390                 395                 400

Ser Ser Ser Asp Pro His Gly Ile Glu Ser Lys Arg Arg Lys Lys Lys
                405                 410                 415

Pro Val Phe Leu Asp His Phe Leu Pro Gly His Ser Ser Gly Leu Phe
            420                 425                 430

Leu Pro Pro Ser Ala Leu Gln Pro Ala Asp Ser Asp Phe Phe Pro Ala
        435                 440                 445

Ser Ile Ser Leu Pro Gly Leu Glu Pro Pro Gly Gly Pro Asp Leu Leu
    450                 455                 460

Asp Asp Gly Phe Ala Tyr Asp Pro Ser Ala Pro Thr Leu Phe Thr Met

465                 470                 475                 480

Leu Asp Leu Leu Pro Pro Ala Pro Pro Leu Ala Ser Ala Val Val Gly
                485                 490                 495

Ser Gly Gly Ala Gly Ala Thr Val Val Glu Ser Ser Gly Pro Glu Pro
            500                 505                 510

Leu Ser Leu Asp Ser Phe Ala Ala Pro Gly Pro Gly Asp Val Gly Thr
        515                 520                 525

Ala Ser Leu Val Gly Ser Asn Met Phe Pro Asn Gln Tyr Arg Glu Ala
    530                 535                 540

Ala Phe Gly Gly Gly Leu Leu Ser Pro Gly Pro Glu Ala Thr
545                 550                 555

<210>  2
<211>  747
<212>  PRT
<213>  Homosapiens

<400>  2
   Met Ala Asp Glu Ala Ala Leu Ala Leu Gln Pro Gly Gly Ser Pro Ser
   1 5 10 15
   Ala Ala Gly Ala Asp Arg Glu Ala Ala Ser Ser Pro Ala Gly Glu Pro
   20 25 30
   Leu Arg Lys Arg Pro Arg Arg Asp Gly Pro Gly Leu Glu Arg Ser Pro
   35 40 45
   Gly Glu Pro Gly Gly Ala Ala Pro Glu Arg Glu Val Pro Ala Ala Ala
   50 55 60
   Arg Gly Cys Pro Gly Ala Ala Ala Ala Ala Leu Trp Arg Glu Ala Glu
   65 70 75 80
   Ala Glu Ala Ala Ala Ala Gly Gly Glu Gln Glu Ala Gln Ala Thr Ala
   85 90 95
   Ala Ala Gly Glu Gly Asp Asn Gly Pro Gly Leu Gln Gly Pro Ser Arg
   100 105 110
   Glu Pro Pro Leu Ala Asp Asn Leu Tyr Asp Glu Asp Asp Asp Asp Glu
   115 120 125
   Gly Glu Glu Glu Glu Glu Ala Ala Ala Ala Ala Ile Gly Tyr Arg Asp
   130 135 140
   Asn Leu Leu Phe Gly Asp Glu Ile Ile Thr Asn Gly Phe His Ser Cys
   145 150 155 160
   Glu Ser Asp Glu Glu Asp Arg Ala Ser His Ala Ser Ser Ser Asp Trp
   165 170 175
   Thr Pro Arg Pro Arg Ile Gly Pro Tyr Thr Phe Val Gln Gln His Leu
   180 185 190
   Met Ile Gly Thr Asp Pro Arg Thr Ile Leu Lys Asp Leu Leu Pro Glu
   195 200 205
   Thr Ile Pro Pro Pro Glu Leu Asp Asp Met Thr Leu Trp Gln Ile Val
   210 215 220
   Ile Asn Ile Leu Ser Glu Pro Pro Lys Arg Lys Lys Arg Lys Asp Ile
   225 230 235 240
   Asn Thr Ile Glu Asp Ala Val Lys Leu Leu Gln Glu Cys Lys Lys Ile
   245 250 255
   Ile Val Leu Thr Gly Ala Gly Val Ser Val Ser Cys Gly Ile Pro Asp
   260 265 270
   Phe Arg Ser Arg Asp Gly Ile Tyr Ala Arg Leu Ala Val Asp Phe Pro
   275 280 285
   Asp Leu Pro Asp Pro Gln Ala Met Phe Asp Ile Glu Tyr Phe Arg Lys
   290 295 300
   Asp Pro Arg Pro Phe Phe Lys Phe Ala Lys Glu Ile Tyr Pro Gly Gln
   305 310 315 320
   Phe Gln Pro Ser Leu Cys His Lys Phe Ile Ala Leu Ser Asp Lys Glu
   325 330 335
   Gly Lys Leu Leu Arg Asn Tyr Thr Gln Asn Ile Asp Thr Leu Glu Gln
   340 345 350
   Val Ala Gly Ile Gln Arg Ile Ile Gln Cys His Gly Ser Phe Ala Thr
   355 360 365
   Ala Ser Cys Leu Ile Cys Lys Tyr Lys Val Asp Cys Glu Ala Val Arg
   370 375 380
   Gly Asp Ile Phe Asn Gln Val Val Pro Arg Cys Pro Arg Cys Pro Ala
   385 390 395 400
   Asp Glu Pro Leu Ala Ile Met Lys Pro Glu Ile Val Phe Phe Gly Glu
   405 410 415
   Asn Leu Pro Glu Gln Phe His Arg Ala Met Lys Tyr Asp Lys Asp Glu
   420 425 430
   Val Asp Leu Leu Ile Val Ile Gly Ser Ser Leu Lys Val Arg Pro Val
   435 440 445
   Ala Leu Ile Pro Ser Ser Ile Pro His Glu Val Pro Gln Ile Leu Ile
   450 455 460
   Asn Arg Glu Pro Leu Pro His Leu His Phe Asp Val Glu Leu Leu Gly
   465 470 475 480
   Asp Cys Asp Val Ile Ile Asn Glu Leu Cys His Arg Leu Gly Gly Glu
   485 490 495
   Tyr Ala Lys Leu Cys Cys Asn Pro Val Lys Leu Ser Glu Ile Thr Glu
   500 505 510
   Lys Pro Pro Arg Thr Gln Lys Glu Leu Ala Tyr Leu Ser Glu Leu Pro
   515 520 525
   Pro Thr Pro Leu His Val Ser Glu Asp Ser Ser Ser Pro Glu Arg Thr
   530 535 540
   Ser Pro Pro Asp Ser Ser Val Ile Val Thr Leu Leu Asp Gln Ala Ala
   545 550 555 560
   Lys Ser Asn Asp Asp Leu Asp Val Ser Glu Ser Lys Gly Cys Met Glu
   565 570 575
   Glu Lys Pro Gln Glu Val Gln Thr Ser Arg Asn Val Glu Ser Ile Ala
   580 585 590
   Glu Gln Met Glu Asn Pro Asp Leu Lys Asn Val Gly Ser Ser Thr Gly
   595 600 605
   Glu Lys Asn Glu Arg Thr Ser Val Ala Gly Thr Val Arg Lys Cys Trp
   610 615 620
   Pro Asn Arg Val Ala Lys Glu Gln Ile Ser Arg Arg Leu Asp Gly Asn
   625 630 635 640
   Gln Tyr Leu Phe Leu Pro Pro Asn Arg Tyr Ile Phe His Gly Ala Glu
   645 650 655
   Val Tyr Ser Asp Ser Glu Asp Asp Val Leu Ser Ser Ser Ser Cys Gly
   660 665 670
   Ser Asn Ser Asp Ser Gly Thr Cys Gln Ser Pro Ser Leu Glu Glu Pro
   675 680 685
   Met Glu Asp Glu Ser Glu Ile Glu Glu Phe Tyr Asn Gly Leu Glu Asp
   690 695 700
   Glu Pro Asp Val Pro Glu Arg Ala Gly Gly Ala Gly Phe Gly Thr Asp
   705                 710                 715                 720
   Gly Asp Asp Gln Glu Ala Ile Asn Glu Ala Ile Ser Val Lys Gln Glu
                   725                 730                 735
   Val Thr Asp Met Asn Tyr Pro Ser Asn Lys Ser
               740                 745

<210> 3
<211> 22
<212> DNA
<213> Homo sapiens

<400> 3
caacagtgta cttacgcaga cg                                                22

<210> 4
<211> 22
<212> DNA
<213> Homo sapiens

<400> 4
ctagctgcct gcctcattct ac                                                22

<210> 5
<211> 23
<212> DNA
<213> Homo sapiens

<400> 5
gacatgctca agtgctgagt cac                                               23

<210> 6
<211> 21
<212> DNA
<213> Homo sapiens

<400> 6
agattgcaca atgtgacgtc g                                                 21

<210> 7
<211> 21
<212> DNA
<213> Homo sapiens

<400> 7
tcagtgtcat ggttcctttg c                                                 21

<210> 8
<211> 21
<212> DNA
<213> Homo sapiens

<400> 8
aatctgctcc tttgccactc t                                                 21

<210> 9
<211> 21
<212> DNA
<213> Homoa sapi ens

<400> 9
agt gt agccc aggat gccct t 21


<210> 10
<211> 21
<212> DNA
<213> Homo sapi ens

<400> 10
gccaaggt ca t ccat gacaa c 21

## Description

### Claim of Priority

This application claims priority to U.S. Provisional Patent Application No. 61/016,179, filed December 21, 2007, the disclosure of which is hereby incorporated by reference herein.

### Field of the Invention

The present invention relates to molecular targets for the treatment of inflammation pathologies, such as chronic obstructive pulmonary disease (COPD). Specifically, the present invention relates to the molecular targets RelB and SIRT1, which are involved in certain inflammatory responses. Treatments for inflammation pathologies may be effected by manipulating the levels and function of these factors.

### Background of the Invention

Chronic obstructive pulmonary disease (COPD) is a major cause of disability, morbidity and mortality in smokers. It is characterized by progressive and largely irreversible airflow limitation, which is associated with an abnormal inflammatory response in the lung (55-57). Cigarette smoking is the primary cause of COPD characterized by accelerated decline in lung function and alveolar destruction of the lung (57, 58). The inflammatory changes that occur in COPD are also seen in cigarette smokers without COPD, but to a lesser extent (59, 60). COPD is linked with the aging process of the lungs due to its direct encounter with inhaled cigarette smoke (CS)-derived oxidants and free radicals and other organic constituents (58, 61). It is well known that abnormal inflammatory responses to smoking in subjects with COPD are due to chronic inflammatory effects incited by CS (62). However, due to the complex nature of the mechanism(s) of the inflammatory processes in COPD (60, 62), the precise molecular mechanisms whereby CS triggers abnormal and sustained lung inflammation and aging process still remains unclear.

However, it is known that CS is a major etiologic factor in the pathogenesis of COPD (1). The present inventors, and others, have shown that CS exposure resulted in lung inflammation with an increased in inflammatory cell influx, such as macrophages, neutrophils, CD8+ lymphocytes, and increased release of pro-inflammatory mediators (2-10). The numbers of neutrophils, macrophages, and lymphocytes have been shown to be increased in both airways and parenchyma of subjects with COPD (11).

Recently, it has been reported that the progression and severity of COPD is associated with increasing infiltration of airways by innate and adaptive inflammatory immune cells, such as polymorphonuclear leukocytes, macrophages, lymphocyte subtypes CD4+, CD8+ and B-cells which are accumulated in absolute volume in the pool of inflammatory cells and form lymphoid follicles (12, 13). These lymphoid cells, such as CD4+, CD8+ and B-cells cells are involved in adaptive immune response which is highly specific and has a specific memory to previous insults. Furthermore, these cells are involved in airway obstruction of the small airways and is associated with a thickening of the airway wall in subjects with COPD (12).

Recently, Van der Strate and coworkers have reported that B-cell follicles are detected in lung sections of mice with emphysema and in subjects with emphysema associated with increased release of pro-inflammatory mediators [IL-4, IL-6, KC(IL-8), RANTES, MCP-1, IP-10 and IL-13] (13), suggesting that proliferating B-cells contribute to the inflammatory process in the aggregates of lymphoid follicles and/or development and perpetuation of emphysema. However, the presence of these B-cell follicles in CS-mediated nuclear factor-kappaB (NF-κB)-driven inflammatory process, specific antigen-driven reaction, or antibody production in the pathogenesis of COPD is not known.

The NF-κB family of transcription factors is essential for the control of immune and inflammatory response as well as cell survival, proliferation and differentiation. The classical pathway requires IκB-kinase (IKKβ) activity, whereas the alternative pathway involves selective nuclear translocation of p52:RelB dimer upon NF-κB-inducing kinase (NIK)-mediated phosphorylation of IKKα. The present inventors have recently shown that RelA/p65 subunit of NF-κB is activated in response to CS and oxidants leading to increased release of inflammatory mediators which are involved in airway inflammation and pathogenesis of COPD (4, 14-19). The alternative NF-κB pathway is important in lymphoid organogenesis, B-lymphocyte differentiation, immune response and antibody production (20-23). Furthermore, it has been also shown that RelB inhibits NF-κB-dependent pro-inflammatory mediator gene expression and RelB is inducibly degraded upon activation of lymphoid cells (24, 25), whereas activation of RelB leads to proinflammatory cytokines release in non-lymphoid cells (21). Moreover, IKKα plays a critical role in activation of RelB/p52 pathway by processing p100 to form p52, which then associates predominantly with RelB in the cytoplasm (23, 26-28). The role of RelB and its signaling pathway in response to environmental agents, particularly in response to CS in different immuneinflammatory and lymphoid cell-types are not known. +

The metabolic nicotinamide adenine dinucleotide (NAD⁺)-dependent protein deacetylases have emerged as important regulators of chronic inflammatory diseases, cancer and aging (63). These proteins, which belong to class III histone/protein deacetylases (HDACs), are referred to as sirtuins. The founding member yeast Sir2 (ySir2, yeast silent information regulator 2), homologous to human sirtuinl (SIRT1), is essential for maintaining silent chromatin via the deacetylation of histones. Activation or overexpression of SIRT1 has been shown to increase the lifespan of fly-Drosophila, *yeast-Saccharomyces cerevisiae,* worm-*Caenorhabditis elegans* (up to 70%), and mouse strain-C57BL/6J (64-69). Recently, it has been shown that SIRT1 plays an important role in a wide variety of processes including stress resistance, metabolism, skeletal muscle dysfunction, apoptosis, senescence, differentiation and aging (63).

SIRT1 negatively regulates transcription factors, such as NF-κB in the nucleus by the deacetylation of modified lysine residues on histones, transcription factors and other non-histone proteins (70-72). Recently, it has been shown that SIRT1 regulates NF-κB-dependent transcription and cell survival in response to TNF-α and other pro-inflammatory mediators (73). It has been suggested that SIRT1 deacetylase may directly bind to one or more constituents in the chromatin complex resulting in structural reorganization, and therefore has the ability to establish silent chromatin domains (71). Evidence indicates that sirtuins have evolved to mediate signaling initiated by stress conditions such as metabolic alterations-nutritional deprivation and calorie restriction (74). However, environmental stress, such as CS exposure, has been shown to decrease the levels of SIRT1 dramatically both in macrophages *in vitro* and in rat lungs *in vivo.* This reduction has also been found to be associated with increased pro-inflammatory cytokine release (75). However, the regulation of SIRT1 in the lungs of smokers and COPD subjects, and its role in regulation of NF-κB-dependent pro-inflammatory cytokines is unknown.

The pathophysiology of COPD is multifactorial with an inflammatory cell profile that includes macrophages, neutrophils, and T-lymphocytes (62, 76, 77). Macrophages are one of the most predominant inflammatory cell types involved in chronic inflammatory states, such as COPD, since they secrete both neutrophil and macrophage chemotactic factors and related chemokines, and matrix metalloproteases (MMPs) (60, 78). The influx of macrophages in the lungs by cigarette smoking leads to increased expression of pro-inflammatory cytokines (78), which is now recognized to be an outcome of chromatin remodeling due to altered acetylation/deacetylation of histone proteins (79, 80). Hence, the involvement of sirtuin (SIRT1) in the regulation of pro-inflammatory cytokine gene expression by deacetylation of NF-κB subunit(s) in human macrophages and other lung cells appears to be highly possible. Similarly, SIRT1 may regulate skeletal muscle and endothelial dysfunction which are the hallmark in pathogenesis of COPD.

Therefore, there remains a need in the art for methods of treating COPD through molecular targets involved in the pathogenesis of COPD.

### Summary of the Invention

It is an object of the present invention to provide methods for the treatment of inflammatory disorders, such as chronic obstructive pulmonary disorder (COPD), rheumatoid arthritis, asthma, idiopathic pulmonary fibrosis, aging and inflammation-associated lung diseases. The methods of the present invention modify the expression and or functionality of molecular targets RelB and/or SIRT1.

It is an object of the present invention to provide a method for treating a subject with an inflammatory disorder by administering to the subject a medicament having a compound that increases the activity of RelB and at least one pharmaceutically acceptable excipient. The compound may increase the ability of RelB to form a heterodimer with RelA, causing the reduction of inflammation.

It is a further object of the present invention to provide a method for treating a subject with an inflammatory disorder by administering the subject a nucleic acid sequence that encodes an amino acid having a sequence that is substantially similar to the amino acid sequence of wild type RelB. The nucleic acid sequence will cause increased concentrations of RelB in the cells of the subject, causing reduced inflammation.

It is a further object of the present invention to provide a method for increasing the expression of endogenous RelB in the cells of a subject. The endogenous levels of RelB may be increased by modifying the promoter of the RelB gene or by administering to the cells a factor that causes increased expression of RelB.

It is an object of the present invention to provide a method for treating a subject with an inflammatory disorder by administering to the subject a medicament having a compound that increases the activity of SIRT1 and at least one pharmaceutically acceptable excipient. The compound may increase the histone deacetylase activity of SIRT1.

It is a further object of the present invention to provide a method for treating a subject with an inflammatory disorder by administering the subject a nucleic acid sequence that encodes an amino acid having a sequence that is substantially similar to the amino acid sequence of wild type SIRT1. The nucleic acid sequence will cause increased concentrations of SIRT1 in the cells of the subject, causing reduced inflammation.

It is a further object of the present invention to provide a method for increasing the expression of endogenous SIRT1 in the cells of a subject. The endogenous levels of SIRT1 may be increased by modifying the promoter of the SIRT1 gene or by administering to the cells a factor that causes increased expression of SIRT1.

**Detailed Description of the Drawings**

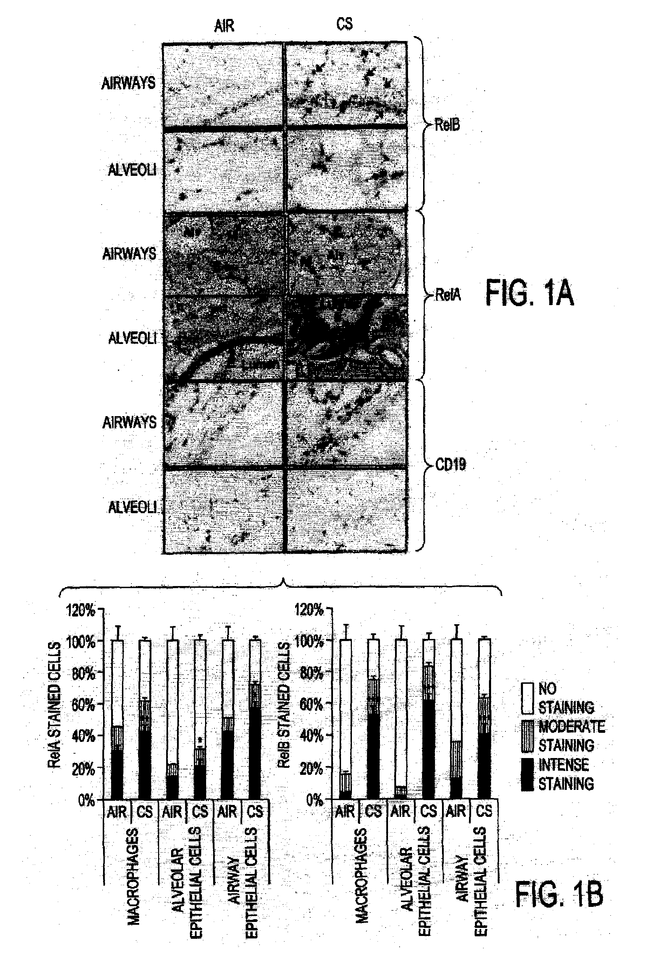

Figure 1. CS exposure increased the levels of RelB in alveolar macrophages and alveolar/airway epithelial cells but not in CD19+ B cells. Mice were exposed to CS for 3 days, and were killed at 24 h of post-last CS exposure. (A) Representative photographs (400×) from immunostaining for RelB, RelA and CD19 in lung tissues from air- and CS-exposed mice. Appearance of dark brown color represents the presence of RelB and RelA, which were increased in various mouse lung cells in response to CS exposure. Arrows indicate RelB- and RelA-positive macrophages, type II- and airway epithelial- cells in mouse lung. However, the expression of CD19 (B-cells) was not altered in lungs. Four slides of each sample of mouse lung tissue were used for immunohistochemistry. E-Epithelial cells; MMacrophages; Alv-Alveoli. (B) Immunostaining scores showing RelB and RelA per cell type in alveolar and airway regions of the lung. The assessment of immunostaining intensity was performed semi-quantitatively in a blinded fashion. Black bars-Intense staining; Grey bars-Moderate/weak staining; White bars-No staining. Results are mean of 5 experiments ± SEM (n=5). *p<0.05, **p<0.01, and ***p<0.001, significantly different from respective air exposed groups.

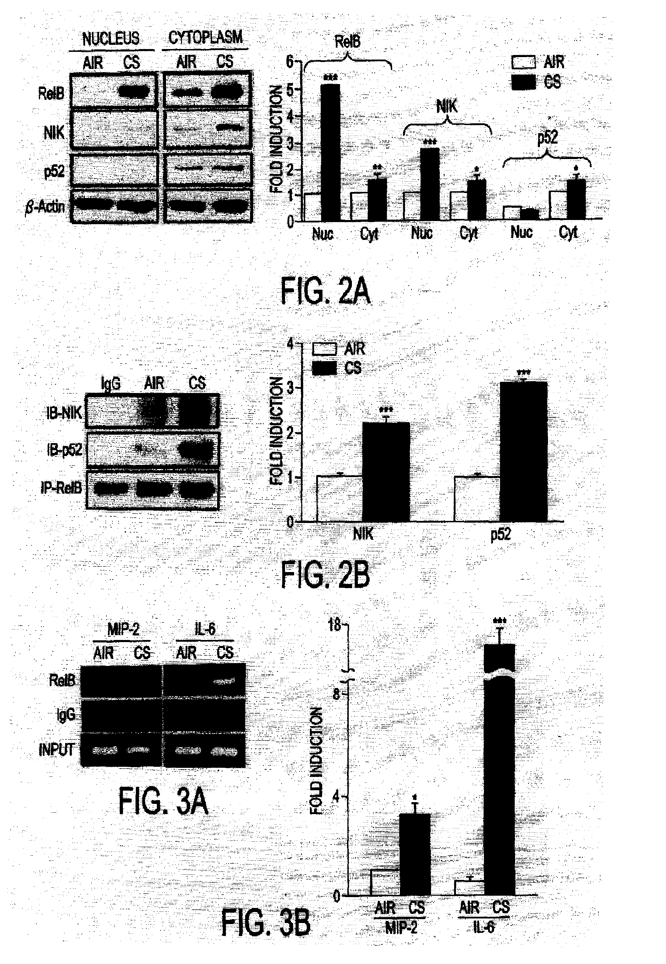

Figure 2. CS exposure increased the level of RelB and induced the interaction of RelB with NIK and p52 in mouse lung.Mice were exposed to CS for 3 days, and were killed at 24 h of post-last CS exposure. (A) The levels of RelB and NIK were significantly increased in both nucleus and cytoplasm of mouse lung tissue, whereas the level of p52 was increased only in cytoplasm but not in the nucleus in response to CS. β-actin was used as a loading control. (B) The level of RelB, and its interaction with NIK and p52, in lungs was increased in response to CS (fold induction vs. control). Nuc: nucleus and Cyt: cytoplasm. Data are shown as mean ± SEM, *p<0.05, **p<0.01, and ***p<0.001 significantly different from respective air-exposed mice (n=4/group).

Figure 3. CS exposure led to recruitment of RelB on promoters of IL-6 and MIP-2 genes in mouse lung. Mice were exposed to CS for 3 days and killed at 24 h post-last CS exposure. (A) CS exposure caused recruitment of RelB on MIP-2 and IL-6 promoters. The nuclear extracts were immunoprecipitated with specific antibodies, and binding to the promoters of proinflammatory mediator genes was detected by PCR-primers for IL-6 and MIP-2. Binding to the promoters is compared with PCR of the input DNA. IgG was used as a negative control. (B) The bands were measured by densitometry (fold induction vs. control). Data are shown as mean ± SEM, *p<0.05, and **p<0.01, significantly different from respective air-exposed mice (n=4/group).

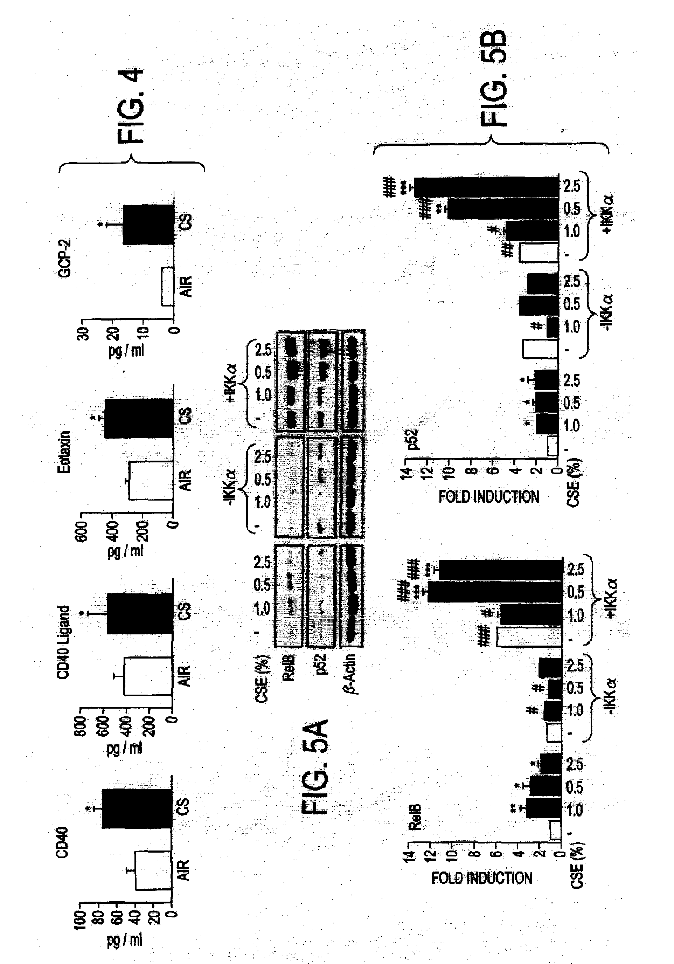

Figure 4. CS exposure increased the levels of RelB-dependent pro-inflammatory mediators in mouse lung. The levels of RelB-dependent pro-inflammatory cytokines were examined in BAL fluid of mice exposed to CS for 3 days (24 h post-last exposure) by multiplex Luminex-100. Data are shown as mean ± SEM (n=5-6 per group). *p<0.05, significant compared with air-exposed group.

Figure 5. CSE increased the levels of RelB and p52, and loss of IKKα attenuated RelB in human monocyte/macrophages. (A) MonoMac6 cells were transfected with dominant negative and wild-type IKKα plasmids, and treated with CSE (0.5%, 1.0% and 2.5%) for 1 h; and then the protein levels of RelB and p52 were measured by western blotting. CSE increased the levels of RelB and p52 rapidly in nontransfected and untreated cells, whereas the level of RelB was attenuated in cells transfected with dominant negative IKKα plasmids. Transfection of wild-type IKKα increased the levels of RelB and p52. (B) The bands were measured by densitometry (fold induction vs. control). Data are shown as mean ± SEM of n=4 experiments, *p<0.05, **p<0.01 and ***p<0.001, significantly different from respective controls; #p<0.05, ##p<0.01, and ###p<0.001 significantly different from respective non-transfected groups.

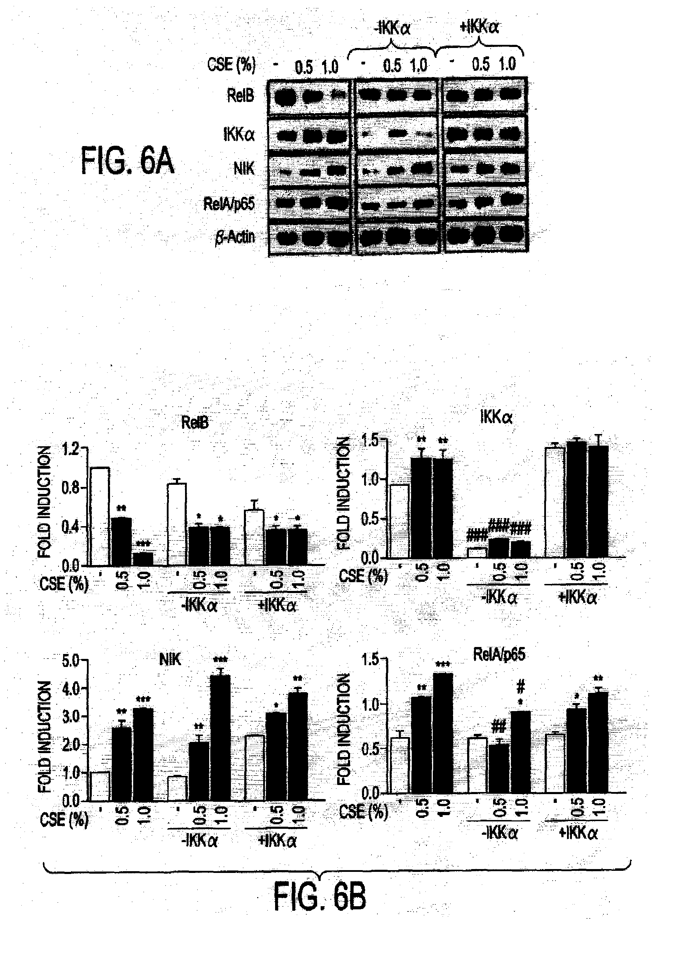

Figure 6. CSE rapidly degraded RelB, and the loss of IKKα partially restored RelB in human B-cells. Ramos B-cells were transfected with dominant negative IKKα plasmid, and treated with CSE (0.5% and 1.0%) for 1 h; and the protein levels of RelB, IKKα, NIK and RelA/p65 were measured by western blotting. The level of RelB was significantly decreased in response to CSE, whereas the levels of IKKα, NIK and RelA/p65 were significantly increased. However, the level of RelB was partially restored in cells transfected with dominant negative IKKα plasmid. Furthermore, the level of RelB was partially attenuated whereas the levels of NIK and RelA/p65 were normalized in cells transfected with wild-type IKKα plasmid. (B) The bands were measured by densitometry (fold induction vs. control). Data are shown as mean ± SEM of n=4 experiments, *p<0.05, **p<0.01 and ***p<0.001, significantly different from respective controls; #p<0.05, ##p<0.01, and ###p<0.001 significantly different from respective non-transfected groups.

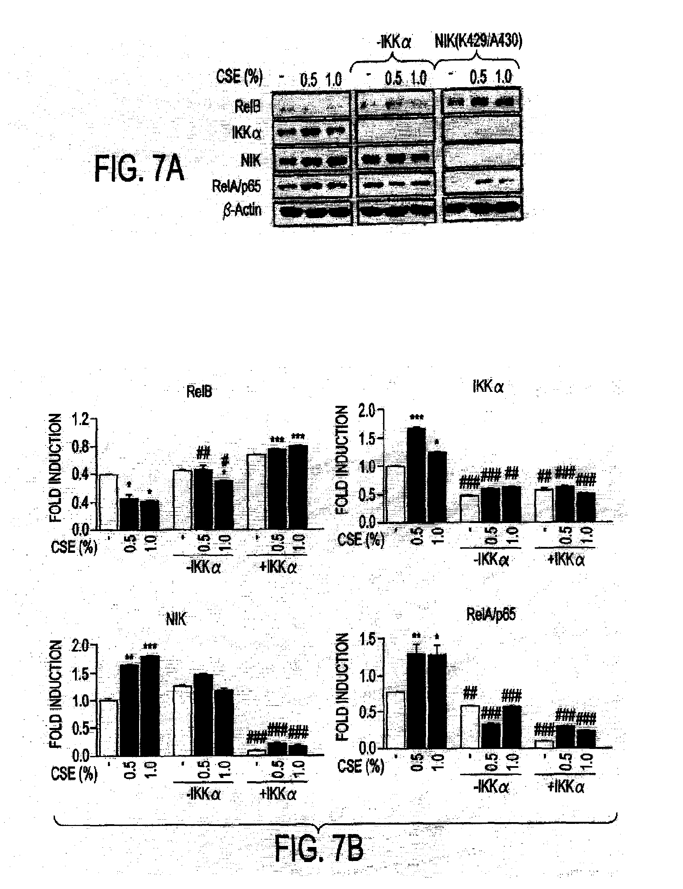

Figure 7. CSE rapidly degraded RelB, and the loss of IKKα and NIK restored RelB in mouse B cell. Mouse immature WEHI-231 B cells were transfected with dominant negative IKKα- and double mutant of NIK (K429/A430)- plasmids, and treated with CSE (0.5% and 1.0%) for 1 h; and the protein levels of NIK, IKKα, RelB and RelA/p65 were measured by western blotting. CSE reduced the levels of RelB, and increased the levels of NIK and IKKα in non-transfected cells, whereas the CSE-mediated reduction in level of RelB was partially attenuated when the cells were transfected with dominant negative IKKα- and completely restored in double mutant of NIK (K429/A430)- plasmids transfected cells. The level of RelA/p65 was increased in nontransfected cells in response to CSE. However, the levels of RelA/p65 and NIK were attenuated in cells transfected with dominant negative IKKα- and double mutant of NIK (K429/A430)- plasmids. β-actin was used as a loading control. (B) The bands were measured by densitometry (fold induction vs. control). Data are shown as mean ± SEM of n=4 experiments, *p<0.05, **p<0.01 and ***p<0.001, significantly different from respective controls; #p<0.05, ##p<0.01, and ###p<0.001 significantly different from respective non-transfected groups.

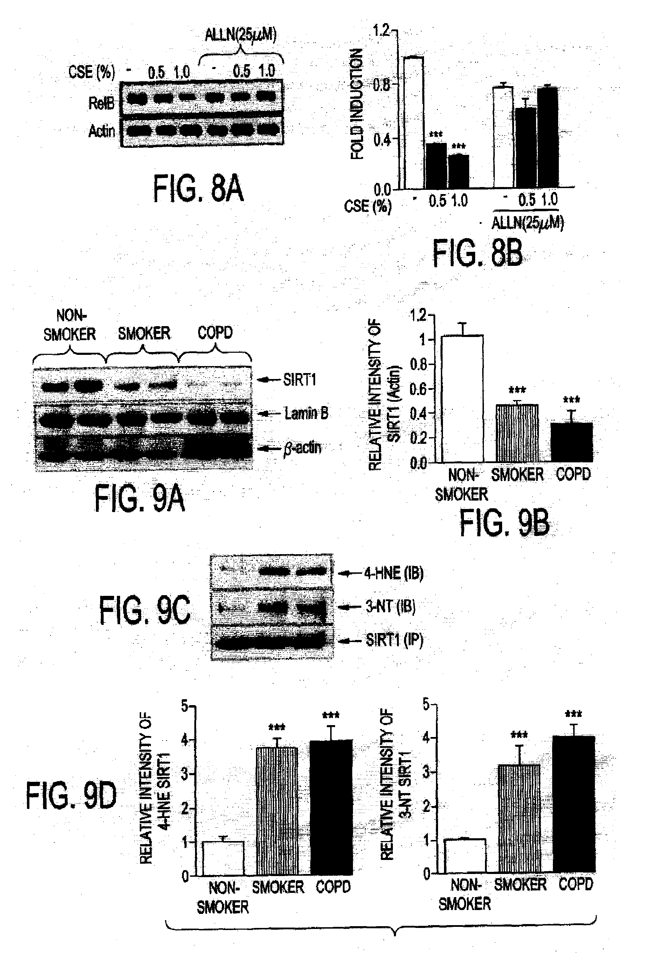

Figure 8. CSE decreased the level of RelB via proteasome-mediated degradation in B-cells. (A) CSE dose-dependently decreased the levels of RelB in B-cells, and the proteasome inhibitor,ALLN, prevented the degradation of RelB by proteasome-dependent mechanism. The cells were pretreated with the proteasome inhibitor ALLN (25 µM) for 20 min before exposing to CSE (0.5% and 1.0%) for 1h; and then the level of RelB in whole cell lysates was measured by immunoblotting. (B) The bands were measured by densitometry (fold induction vs. control). Data are shown as mean ± SEM of n=4 experiments, ***p<0.001, significantly different from respective controls.

Figure 9 Decreased levels of sirtuin (SIRT1) protein in lung tissue of smokers and patients with COPD. A) Western blot analysis of SIRT1 in soluble nuclear proteins (30 µg) extracted from the lung tissue of non-smokers (n=10), smokers (n=10) and COPD patients (n=17). The proteins were electrophoresed on a 7.5% PAGE gel and electroblotted onto a nitrocellulose membrane. The level of SIRT1 protein was determined using mouse monoclonal SIRT1 antibody. The purity of nuclear extract was shown by the presence of lamin B (nuclear envelope protein) and the absence of the cytoskeletal protein α-tubulin (not shown). B) After densitometric analysis, the values were normalized against the loading control, β-actin. The relative level (% of control) of SIRT1 showed decreased levels of nuclear SIRT1 protein in the lung tissues of smokers and COPD patients. C) SIRT1 protein was immunoprecipitated from the nuclear extract of lung homogenates. The levels of SIRT1 adducts with 4-hydroxy-2-nonenol (4-HNE) and nitration of tyrosine residues on SIRT1 were analyzed by immunoblotting with anti-4-HNE and anti-3-nitrotyrosine (3-NT) antibodies, respectively. Equal amount of immunoprecipitated SIRT1 protein (100 µg) was used for Western blotting. D) Relative intensity of 4-HNE/SIRT1 and 3-NT/SIRT1 represents the increased post-translational modifications of SIRT1 protein in lungs of smokers and patients with COPD compared to non-smokers. A representative blot is shown which was obtained from several blotting experiments. Results are expressed as mean ± SEM. ***p<0.001, significant compared to non-smokers.

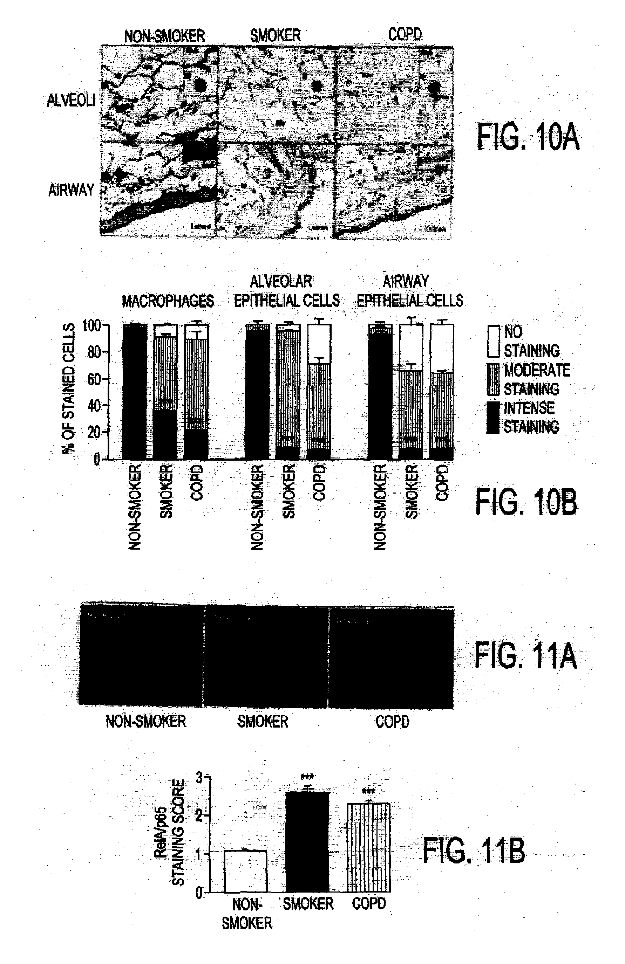

Figure 10 Decreased staining of SIRT1 in lung macrophages and alveolar/airway epithelial cells of smokers with and without COPD. A) Representative photographs (400X) from immunostaining for SIRT1 in lung tissues from non-smokers (n=10) and smokers with (n=17) and without (n=10) COPD. The levels of SIRT1 were measured in the fixed lung sections (3-µm thick) by immunohistochemical staining using SIRT1 rabbit polyclonal antibody (Ab, 1:100 dilution) with avidin-biotin-peroxidase complex (ABC) method followed by hematoxylin counter staining. Appearance of dark brown color represents the presence of SIRT1 (indicated with thick arrow), which was decreased in smokers' lung (indicated with thin arrow). E- Epithelial cells; M-Macrophage; Alv-Alveoli; Aw-Airway. B) Immunostaining scores for SIRT1 per cell type in alveolar and airway regions of the lung. The assessment of immunostaining intensity was performed semi-quantitatively and in a blinded fashion. Black bars-Intense staining; Gray bars-Moderate/weak staining; White bars-No staining. Results are represented as mean ± SEM. ***p<0.001, significant compared to non-smokers.

Figure 11 Decreased levels of SIRT1 in smokers and COPD patients were associated with increased levels of RelA/p65 NF-κB. The expression of NF-κB was measured in fixed lung sections (3-µm thick) of non-smokers (n=10), and smokers with (n=17) and without (n=10) COPD by immunohistochemical staining using anti-RelA/p65 NF-κB antibody (1:100 dilution) followed by incubations with FITC conjugated anti-rabbit secondary antibody. Representative immunoflourescent images (400X) showed increased levels of NF-κB in the lungs (especially in macrophages and epithelial cells) of smokers with and without COPD as compared to non-smokers. Arrows indicate the cells (airway/alveolar epithelial cells and macrophages) that express increased levels of RelA/p65 NF-κB proteins. B) Immunostaining score for RelA/p65 was performed semi-quantitatively and in a blinded fashion: 0 = No staining, 1 = weak staining, 2 = Moderate staining, 3 = Intense staining. Results are expressed as mean ± SEM. ***p<0.001, significant compared to non-smokers.

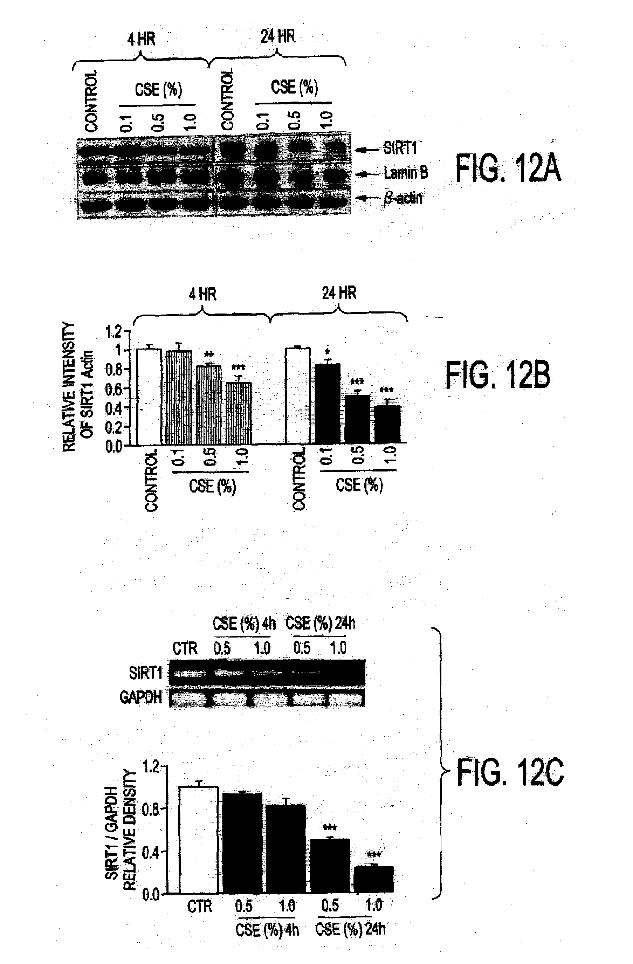

Figure 12 Decreased levels of SIRT1 protein and mRNA expression by CSE treatment in MonoMac6 cells. A) Western blots of soluble nuclear proteins (30 µg) extracted from CSE-exposed MonoMac6 cells. Expression of SIRT1 was determined using mouse monoclonal SIRT1 antibody. The purity of nuclear extract was shown by the presence of lamin B (nuclear envelope protein) and the absence of the cytoskeletal protein α-tubulin (bands not shown). Gel pictures shown are representative of at least three separate experiments. B) Densitometric values of SIRT1 were normalized against the loading control, β-actin. The relative level (% of control) of SIRT1 in MonoMac6 cells showed decreased levels of SIRT1 protein in response to CSE at 4 and 24 hr. C) CSE decreased the levels of SIRT1 mRNA in MonoMac6 cells. After 4 and 24 h of CSE treatment, total RNA was extracted from monocyte-macrophage cells (MonoMac6) using RNeasy kit (Qiagen). Reverse transcriptase-polymerase chain reaction (RT-PCR) was performed. Amplified products (SIRT1:200 bp; GAPDH:600 bp) were resolved by 1.5% agarose gel electrophoresis, stained with ethidium bromide. SIRT1 mRNA expression was decreased following 24 h exposure to low concentrations of CSE (0.5% and 1%) compared to control. Data represent mean ± SEM of 3 experiments (n=3). *p<0.05, **p<0.01, ***p<0.001, significant compared to control values.

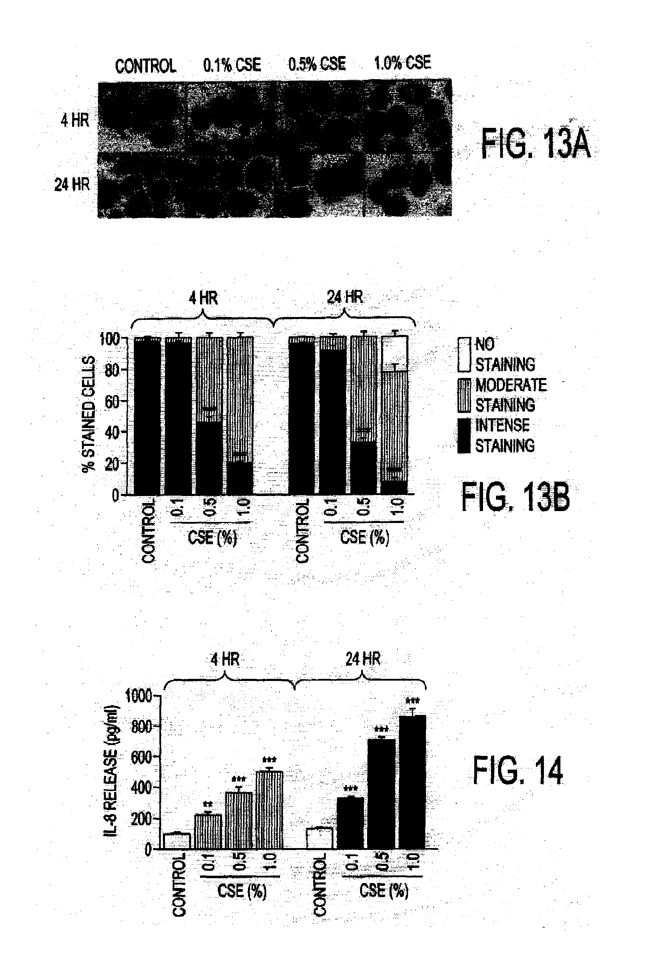

Figure 13 Decreased SIRT1 protein staining in response to CSE treatment in MonoMac6 cells. A) CSE decreased the levels of SIRT1 in MonoMac6 cells at 4 and 24 hr. MonoMac6 cells were treated with different concentrations of CSE (0.1-1.0%). Cells were harvested and the cytospin slides were prepared at 4 and 24 hr of treatments. Immunostaming was performed using a rabbit polyclonal antibody specific for SIRT1 followed by the avidin-biotin-peroxidase complex (ABC) method, and counterstained with hematoxylin. Appearance of dark brown color represents the presence of SIRT1, which was decreased in response to CSE treatment. B) Graph showing the percentage (%) of SIRT1 positive cells from the total number of cells in CSE-treated MonoMac6 cells. The assessment of immunostaining intensity was performed semi-quantitatively and in a blinded fashion. Black bars-Intense staining; Gray bars-Moderate/weak staining; White bars-No staining. Results shown are means ± SEM of three separate experiments (n=3). ***p<0.001, significant compared to control values.

Figure 14 CSE induced IL-8 release from MonoMac6 cells. MonoMac6 cells were treated with freshly prepared CSE (0.1,0.5, and 1.0%) for 4 and 24 hr. IL-8 release was measured in the culture media by sandwich ELISA duo-antibody kit (R&D Systems, Minneapolis, MN). CSE showed increase in the levels of IL-8 as compared to controls at 4 and 24 hr. Each histogram represents the mean ± SEM of 3 experiments (n=3). **p<0.01, ***p<0.001, significant compared to controls.

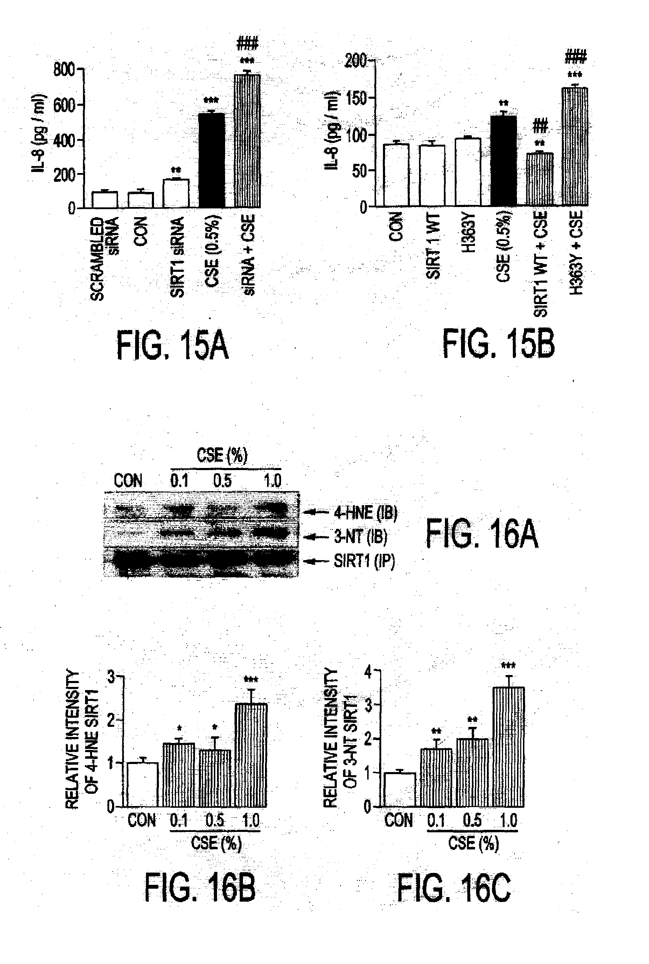

Figure 15 CSE-mediated IL-8 release was modified by SIRT1 knock-down, mutation and overexpression in MonoMac6 cells. A) MonoMac6 cells were transfected with predesigned human SIRT1 siRNA or scrambled non-target siRNA using DharmaFect2 siRNA transfection reagent. After 36-48 hr of transfection (cells >85% viable), the cells were treated with CSE (0.5%) for 12 hr. At the end of the experiment, culture media was collected by centrifugation for IL-8 assay. SIRT1 knock-down led to significant increase in IL-8 release in response to CSE treatment in MonoMac6 cells as compared to non-target scrambled siRNA. B) MonoMac6 cells were transfected with SIRT1 overexpressing plasmid or SIRT1-H363Y (mutated in the deacetylase domain) using calcium phosphate method. Overexpression of SIRT1 deceased IL-8 release whereas SIRT1-H363Y lacking SIRT1 deacetylase domain increased IL-8 release in response to CSE treatment at 4 hr. Each value is the mean ± SEM of triplicate determinations (n=3). **p<0.001, ***p<0.001, significant compared to control; ^{##}p<0.01, ^{###}p<0.001, significant compared to CSE treated group.

Figure 16 CSE caused post-translational modifications of SIRT1. A) SIRT1 protein was immunoprecipitated from the nuclear extract of MonoMac6 cells treated with CSE (0.1, 0.5, and 1.0%) for 4 hr. The levels of SIRT1 adducts with 4-hydroxy-2-nonenol (4-HNE) and nitration of tyrosine residues on SIRT1 were analyzed by immunoblotting with anti-4-HNE and anti-3-nitrotyrosine (3-NT) antibodies, respectively. Equal amount of immunoprecipitated SIRT1 protein (100 µg) was used for Western blotting. Relative intensity of 4-HNE/SIRT1 (B) and 3-NT/SIRT1 (C) represents the increased post-translational modifications of SIRT1 protein in response to CSE treatment. Results are means ± SEM of three separate experiments (n=3). Significant differences are shown from controls: *p<0.05, **p<0.01, and ***p<0.001.

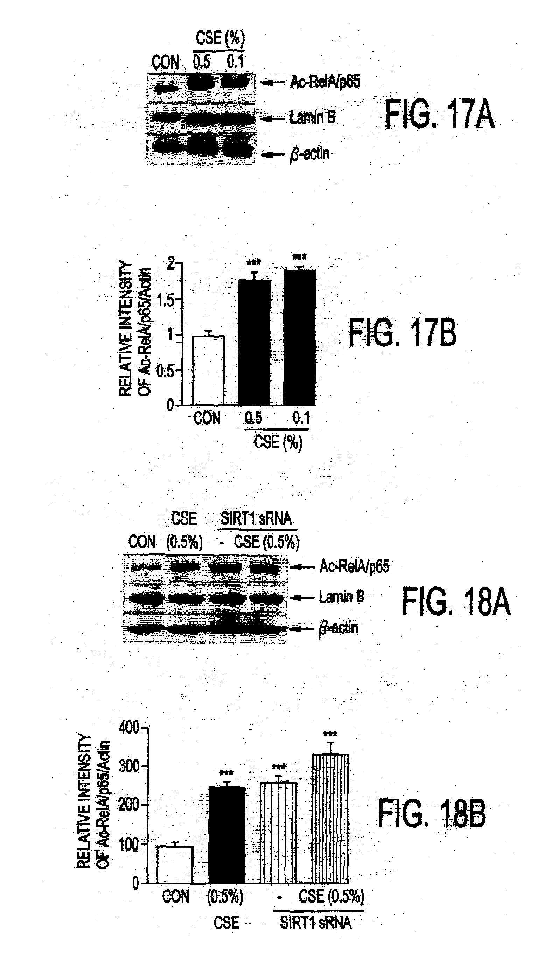

Figure 17 CSE-mediated decrease in SIRT1 level was associated with increased acetylation of RelA/p65 NF-κB. A) MonoMac6 cells were treated with CSE (0.5 and 1.0%) for 4 hr. Acetylation of the lysine residue (K310) on RelA/p65 NF-κB protein was determined in soluble nuclear proteins (30 µg) by Western blotting using anti-acetyl RelA/p65 (K310) antibody. β-action was measured as a loading control. Lamin B (nuclear envelope protein) and the absence of the cytoskeletal protein α-tubulin (bands not shown) were measured to confirm the purity of nuclear extracts. B) The relative density (% of control) of acetylated RelA/p65 NF-κB in nuclear fraction of MonoMac6 cells showed increased acetylation of RelA/p65 NF-κB in response to CSE treatment at 4 hr. Each histogram represents the means ± SEM (n=3). ***p<0.001, compared to control values.

Figure 18 siRNA silencing of SIRT1 augmented the CSE-mediated acetylation of RelA/p65 NF-κB. MonoMac6 cells were transfected with predesigned human SIRT1 siRNA duplex (100 nM) using DharmaFect2 transfection reagent for 36-48 hr and then treated with CSE (0.5%) for 12 hr. siCONTROL non-targeting scrambled siRNA was used as a negative control. Actin was measured as a loading control. A) Acetylation of RelA/p65 NF-κB was determined using anti-rabbit Ac-RelA/p65 (K310) antibody in the soluble nuclear extract. The purity of nuclear extract was shown by the presence of lamin B (nuclear envelope protein) and the absence of the cytoskeletal protein α-tubulin (bands not shown). B) The relative level (% of control) of Ac-RelA/p65 showed increased acetylation of nuclear RelA/p65 in response to SIRT1 knock-down and/or CSE treatment. Each value is the mean ± SEM of triplicate determinations (n=3). ***p<0.001, significant compared to control.

### Detailed Description of the Invention

The present invention provides methods and molecular targets for the treatment of chronic obstructive pulmonary disease (COPD) and other inflammatory diseases including skeletal muscle and endothelial dysfunctions which are associated with COPD. By modification of the levels and/or functionality of the molecular targets, the inflammatory pathways that are part of the pathogenesis of COPD can be controlled or halted. The inflammatory pathways that are controlled by the methods of the present invention are typically NF-κB controlled pathways.

In certain embodiments of the present invention, the molecular target for treatment of COPD is RelB. RelB is one of the five proteins in the mammalian NF-κB transcription factor family, and is capable of forming transcription activating heterodimers with specific other members of the family. The amino acid sequence of wild type human RelB protein is listed as SEQ ID NO. 1.

In certain methods of the present invention, the amount and/or activity of RelB is regulated in a subject having COPD. In certain embodiments, levels of RelB in certain cells of the subject may be either increased or decreased. It is further contemplated that RelB levels may be increased in certain cells while RelB levels are decreased in other cells of the subject.

In certain embodiments of the present invention, subjects are administered compounds that are activators of RelB. The RelB activator compounds may be small molecule compounds, such as small molecule pharmaceuticals, or may also be biological macromolecules such a proteins, peptides and nucleic acids.

The RelB activator compounds may stimulate the production of RelB by stimulating the transcription and/or translation of the *RelB* gene and its transcripts. The RelB activator may also inhibit the elimination of the RelB protein from the cell, either by inhibiting proteolysis of RelB or by inhibiting its transport or translocation. In certain embodiments, RelB activators may inhibit the proteolysis of RelB or may inhibit modifications that target RelB for proteolysis, such as phosphorylation of RelB.

The RelB activator may also stimulate the action of RelB, with or without increasing the level of RelB in the cell. In certain embodiments, the RelB activator may stimulate the formation of RelB heterodimers, may stimulate protein modification of RelB, or may increase the transport of RelB across the nuclear membrane. For example, the RelB activator may stimulate the formation of RelA/RelB heterodimers.

The RelB activators of the present invention may be formulated and delivered to the subject in the same manner as pharmaceutical agents of the same type. For instance, depending on the type of compound, the RelB activators may be delivered to the subject, orally, parenterally, inhalation or topically. The compounds may be formulated using excepients that are well known in the art, including glidants, lubricants, binders, fillers, buffers, pH modifiers and salts. The RelB activators may be administered as frequently as several times a day or as infrequently as a few times a year as necessary.

In certain embodiments of the invention, certain cells of a subject may be caused to produce more endogenous or exogenous RelB. In certain embodiments, cells of the subject are treated using gene therapy methods known in the art to introduce a nucleic acid sequence encoding the RelB protein or a derivative thereof. The nucleic acid may encode a protein having 90% or greater sequence similarity to SEQ ID NO. 1. In other embodiments, the encoded protein may have 95% or greater sequence similarity to SEQ ID NO. 1. In still further embodiments, the encoded protein may have 98% or greater sequence similarity to SEQ ID NO. 1.

The nucleic acid sequence may be administered to the cell as part of a vector or other nucleic acid that allows for the integration of the nucleic acid sequence into the host cell's chromosome. It is further contemplated that the nucleic acid sequence be administered to the cell using a extrachromosomal vector that does not integrate into the chromosome.

The nucleic acid sequences encoding RelB and its derivatives may be administered to the subject using gene therapy methods that are well known in the art. In certain embodiments, the nucleic acid sequences are administered using viral vectors. In other embodiments, cells may be removed from the subject to be treated and then transfected using viral or non-viral methods known in the art, such as naked DNA transfection, electroporation, lipoplexes and polyplexes, and dendrimers. After the nucleic acid sequence is transfected into to the isolated cells, the cells may then be administered the subject using known methods.

It is contemplated that exogenous RelB mutants may be administered to subjects. These RelB mutants may contain amino acid changes that provide them with enhanced transcription factor activity. For example, RelB mutants which interact strongly with RelA/p65. Preferably the RelB mutants having enhanced transcription factor activity will bind more tightly to RelA/p65 than wild type RelB. In certain embodiments of the invention, serine 368 of SEQ ID NO. 1 is mutated to enhance the binding of RelB to RelA/p65. It is also contemplated that other residues surrounding serine 368, such as residues 350 - 380, may be mutated in order to enhance the binding of RelB to RelA/p65.

It is also contemplated that RelB mutants may be introduced which are resistant to protein degradation. Such degradation resistant mutants may have mutations at sites that are typically modified to "mark" the protein for degradation. For example, certain threonine or serine residues, or tyrosine residues, which are substrates for protein kinases, may be changed to residues that cannot be phosphorylated. Typically, such residues will be substituted with alanine, although other substitutions are contemplated. In certain embodiments of the invention, residues threonine 84 and serine 552 are mutated to prevent phosphorylation at those sites. It is also contemplated that RelB mutants lacking entire domains may be used.

In certain embodiments of the present invention, exogenenous RelB may be delivered using the above methods to only specific cell types. In certain embodiments, exogenous RelB is delivered to lymphoid cells, such as B-cells and T-cells in the lung. Delivery to specific cell types may be effected using specific viral vectors, or by the isolation and treatment of the specific cell types, followed by re-administration to the subject, both of which are well known in the art.

It is also contemplated that endogenous levels of RelB may be controlled. In certain embodiments, the native RelB promoter may be replaced using homologous recombination methods with a promoter that causes greater production of transcript from the *RelB* gene.

In methods where levels of RelB are increased in cells, it may be necessary to co-administer a RelA inhibitor or IKK2 inhibitor to the subject. An example of an IKK2 inhibitor is SC-514, sold by Merck Senono of Geneva, Switzerland; BAY 11-7085 sold by Calbiochem of Gibbstown, NJ and IMD-0354 sold by Sigma of St. Louis, MO. Coadministration of an RelA or IKK2 inhibitor will help to prevent any unwanted side effects that may be caused by increased RelB levels in certain cells.

As an alternative embodiment, the amount of RelB in cells may be downregulated, for example through promoter modification or through using of antisense nucleic acids as is well known in the art.

The downregulation of RelB may be effected only in specific cell types to obtain a desired effect. In certain embodiments, the levels of expression of RelB may be downregulated in lung cells and macrophages. Specific cell types may be targeted for downregulation of RelB through the use of certain viral vectors or through the specific isolation of certain cell types, followed by treatment of the isolated cells and re-administration of the cells to the subject.

RelB in lung cells including macrophages and lymphoid cells (B and T-cells) can be regulated by nebulizer and/or inhalation devise, nanoparticle formulation using recombinant proteins, mutants, DNA/viral vectors, and in combination with existing therapies including steroids, bronchodilators, β-agonists, antioxidants and/or PDE4 inhibitors.

Without wishing to be bound by theory, it is thought that RelB binds to RelA/p65 to form transcriptionally inactive complexes. This prevents RelA/p65 from binding to κB-sites which are involved in inflammation pathways. As such, COPD and other inflammation-based diseases such as rheumatoid arthritis, asthma and idiopathic pulmonary fibrosis can be treated through targeting of RelB.

In certain embodiments of the present invention, the molecular target for treatment of COPD is SIRT1. SIRT1, also known as Sirtuin 1, is a class III histone/protein deacetylase (HDAC). The amino acid sequence of wild type human SIRT1 protein is listed as SEQ ID NO. 2.

In certain methods of the present invention, the amount and/or activity of SIRT1 is regulated in a subject having COPD. In certain embodiments, levels of SIRT1 in certain cells of the subject may be either increased or decreased.

In certain embodiments of the present invention, subjects are administered compounds that are activators of SIRT1. The SIRT1 activator compounds may be small molecule compounds, such as small molecule pharmaceuticals, or may also be biological macromolecules such a proteins, peptides and nucleic acids.

The SIRT1 activator compounds may stimulate the production of SIRT1 by stimulating the transcription and/or translation of the *Sirt1* gene and its transcripts. The SIRT1 activator may also inhibit the elimination of the SIRT1 protein from the cell, either by inhibiting proteolysis of SIRT1 or by inhibiting its transport. In certain embodiments, SIRT1 activators may inhibit the proteolysis of SIRT1 or may inhibit modifications that target SIRT1 for proteolysis, such as oxidative or nitrosative modifications of SIRT1.

The SIRT1 activator may also stimulate the action of SIRT1, with or without increasing the level of SIRT1 in the cell. In certain embodiments, the SIRT1 activator may stimulate the histone or protein deacetylase activity of SIRT1, or may stimulate protein modification of SIRT1.

The SIRT1 activators of the present invention may be formulated and delivered to the subject in the same manner as pharmaceutical agents of the same type. For instance, depending on the type of compound, the SIRT1 activators may be delivered to the subject, orally, inhalation, parenterally, or topically. The SIRT1 activators may be administered as frequently as several times a day or as infrequently as a few times a year as necessary.

In certain embodiments of the invention, certain cells of a subject may be caused to produce more endogenous or exogenous SIRT1. In certain embodiments, cells of the subject are treated using gene therapy methods known in the art to introduce a nucleic acid sequence encoding the SIRT1 protein or a derivative thereof. The nucleic acid may encode a protein having 90% or greater sequence similarity to SEQ ID NO. 2. In other embodiments, the encoded protein may have 95% or greater sequence similarity to SEQ ID NO. 2. In still further embodiments, the encoded protein may have 98% or greater sequence similarity to SEQ ID NO. 2.

The nucleic acid sequence may be administered to the cell as part of a vector or other nucleic acid that allows for the integration of the nucleic acid sequence into the host cell's chromosome. It is further contemplated that the nucleic acid sequence be administered to the cell using an extrachromosomal vector that does not integrate into the chromosome.

The nucleic acid sequences encoding SIRT1 and its derivatives may be administered to the subject using gene therapy methods that are well known in the art. In certain embodiments, the nucleic acid sequences are administered using viral vectors. In other embodiments, cells may be removed from the subject to be treated and then transfected using viral or non-viral methods known in the art, such as naked DNA transfection, electroporation, lipoplexes and polyplexes, and dendrimers. After the nucleic acid sequence is transfected into to the isolated cells, the cells may then be administered the subject using known methods.

It is contemplated that exogenous SIRT1 mutants may be administered to subjects. These SIRT1 mutants may contain amino acid changes that provide them with enhanced or decreased histone deacetylase activity. It is also contemplated that SIRT1 mutants may be introduced which are resistant to protein degradation. Such degradation resistant mutants may have mutations at sites that are typically modified to "mark" the protein for degradation. For example, certain cysteine, histidine, or lysine residues, which are targets for oxidative modifications such as the formation of 4-hydroxy-2-nonenal (4-HNE), phosphorylation and nitrosative products such as 3-nitrotyrosine, may be changed to residues that cannot be modified to form these types of products. Typically, such residues will be substituted with alanine, although other substitutions are contemplated. Examples of residues which may be targets of oxidative modification include lysine residues 1020 and 1024 of SEQ ID NO. 2, which are present on the active site domain of SIRT1. It is also contemplated that serine 47 of SEQ ID NO. 2 can be mutated to prevent phosphorylation at that site.

In certain embodiments of the present invention, exogenenous SIRT1 may be delivered using the above methods to only specific cell types. In certain embodiments, exogenous SIRT1 is delivered to lymphoid cells, such as B-cells and T-cells. In other embodiments, SIRT1 is delivered to macrophages and lung cells. Delivery to specific cell types may be effected using specific viral vectors, or by the isolation and treatment of the specific cell types, followed by re-administration to the subject, both of which are well known in the art.

It is also contemplated that endogenous levels of STRT1 may be controlled. In certain embodiments, the native SIRT1 promoter may be replaced using homologous recombination methods with a promoter that causes greater production of transcript from the *Sirt1* gene.

SIRT1 in lung cells including macrophages and lymphoid cells (B and T-cells) can be regulated by nebulizer and/or inhalation devise, nanoparticle formulation using recombinant proteins, mutants, DNA/viral vectors, and in combination with existing therapies including steroids, bronchodilators, β-agonists, antioxidants and/or PDE4 inhibitors.

Without wishing to be bound by theory, it is thought that SIRT1 causes the deacetylation of RelA/p65 subunit of NF-κB, inhibiting transcription of genes involved in inflammatory pathways. By inhibiting inflammatory pathways, the treatment of COPD, asthma and other disorders related to inflammation can be treated.

In all of the embodiments of the present invention, it is contemplated that the subjects for treatment using the methods of the present invention are mammals. Although the subjects are preferably humans, it is also contemplated that other mammalian subjects, such as companion animals, may be treated. It is also contemplated that the methods of the present invention may be used *in vitro,* for use in laboratory experiments in cell culture.

Non-limiting examples of methods of the present invention are given below. It should be apparent to one of skill in the art that there are variations not specifically set forth herein that would fall within the scope and spirit of the invention as claimed below.

Examples

Example **1 -** Materials and **Methods - RelB** Experiments

Reagents

Unless otherwise stated, all biochemical reagents used in this study were purchased from SigmaAldrich Inc., (St. Louis, MO). Antibodies used in the studies include the following: β-actin (CP- 01; Oncogene, San Diego, CA), NIK (A-12), RelB (C-19), NF-κBp52 (K-27), RelA/p65, and CD19 (SC-8417, SC-226, SC-298X, SC-372, and SC-8498 respectively; Santa Cruz Biotechnology Inc., Santa Cruz, CA), and IKKα (05-536; Upstate, Charlottesville, VA).

Animals

Adult male C57BL/6J mice (8-10 weeks, 37 ± 1.5 g; Jackson Laboratory, Bar Harbor, ME) were housed in the Inhalation Core Facility of the University of Rochester. The Animal Research Committee of the University of Rochester approved all animal experimental procedures described in this study.

CS exposure

Mice (6 to 8 per group) were used for acute (3 days) CS exposure. The mice were placed in individual compartments of a wire cage which was placed inside an aerated plastic box connected to the smoke source. The CS was generated from 2R4F research cigarettes (TPM concentration 11.7 mg/cigarette, tar 9.7 mg/cigarette, nicotine 0.85 mg/cigarette; University of Kentucky, Lexington, KY). CS exposure was performed according to the Federal Trade Commission protocol (1 puff/min of 2-s duration and 35 ml volume) in an automatic Baumgartner-Jaeger CSM2082i CS machine (CH Technologies, Westwood, NJ).

Mainstream CS was diluted with filtered air and directed into the exposure chamber. The smoke exposure (TPM per cubic meter of air, mg/m3) was monitored in real time with a MicroDust Pro-aerosol monitor (Casella CEL, Bedford, UK) and verified daily by gravimetric sampling. The smoke concentration was set at a nominal value of -300 mg/m3 TPM by adjusting the flow rate of the dilution air (4, 29-33). Sham control animals were exposed only to filtered air in the same manner for the same duration. Mice were received two 1-hour exposures (1 hour apart) per day for 3 days, and were killed at 24 hours post-last exposure. Concentration of carbon monoxide in the CS filled chamber was ∼350 ppm. The dosimetry of carbon monoxide in CS was estimated by measuring the blood carboxyhemoglobin levels. Mice tolerated CS without the evidence of toxicity (carboxyhemoglobin, CoHb levels ∼17 % and no body weight loss).

Tissue harvest and bronchoalveolar lavage **(BAL)**

Mice were injected with 100 mg/kg (body weight) of pentobarbiturate (Abbott laboratories, Abbott Park, IL) intraperitoneally and sacrificed by exsanguinations. The heart and lung were removed en bloc, and the lungs were lavaged three times with 0.5 ml of 0.9% sodium chloride. The lavage fluid was centrifuged, and the cell-free supernatants were frozen at -80°C for ELISA.

**Immunohistochemistry**

The expression and levels of RelB, RelA/p65 and CD19+ B-cells (34) were measured in the fixed mouse lung sections (4-µm thick) by immunohistochemical staining using specific antibodies (1:100 dilution) with avidin-biotin-peroxidase complex (ABC) method followed by hematoxylin counter staining. Appearance of dark brown color represents the presence of RelB, RelA/p65 and B-cells in various areas of lung sections.

**Cell culture**

The human monocyte-macrophage cell line (mature monocytes-macrophages, MonoMac6), which was established from peripheral blood of a subject with monoblastic leukemia (35, 36), were grown in RPMI1640 medium supplemented with 10% FBS, 2 mM L-glutamine, 100 µg/ml penicillin, 100 U/ml streptomycin, 0.1 mM nonessential amino acids, 1 mM sodium pyruvate, 1 µg/ml human holo-transferrin and 1 mM oxaloacetic acid. These cells do not require phorbol myristate acetate (PMA) to differentiate into the macrophages, thus avoiding any stress to the cells. Human Burkitt B lymphoma cells (Ramos B cells), which was established from the ascetic fluid of a 3-year-old boy with American-type Burkitt lymphoma (37), were grown in RPMI 1640 medium supplemented with 5∼10% FBS, 0.1 mM nonessential amino acids, 1 mM sodium pyruvate, 2 mM L-glutamine, 10 mM HEPES, 100 µg/ml penicillin, 100 U/ml streptomycin and 50 µM 2-mercapthoethanol. Mouse immature B cells (WEHI-231) were grown in RPMI 1640 medium supplemented with 10% FBS, 100 µg/ml penicillin, 100 U/ml streptomycin and 50 µM 2-mercapthoethanol (38). The cells were cultured at 37°C in a humidified atmosphere containing 7.5% CO₂.

**Preparation of aqueous CSE**

Research grade cigarettes (1R3F) were obtained from the Kentucky Tobacco Research and Development Center at the University of Kentucky (Lexington, KY). Tar and nicotine contents of 1R3F were 15 mg/cigarette and 1.16 mg/cigarette, respectively. CSE (10%) was prepared by bubbling smoke from one cigarette into 10 ml of culture medium at a rate of one cigarette per 2 minutes as described previously (39), with modifications (4, 15, 40, 41). The pH of the CSE was adjusted to 7.4 and was sterile filtered through a 0.45-µm filter (25-mm Acrodisc; Pall, Ann Arbor, MI). The CSE preparation was standardized by monitoring the absorbance at 320 nm (optical density of 0.74 ± 0.05). The spectral variations observed between different CSE preparations at 320 nm wavelength were found to be within the acceptable limits. CSE was freshly prepared for each experiment and diluted with culture medium containing 1% FBS immediately before use. Control medium was prepared by bubbling air through 10 ml of culture medium supplemented with 1% FBS, adjusting pH to 7.4, and sterile filtered as described for CSE preparation.

**Treatments**

MonoMac6-, Ramos B- and WEHI-231- cells were seeded at a density of less than 1 x 10₆ cells/well (total final volume = 2 ml), grown to ∼80-90% confluency in six-well plates containing RPMI 1640 medium with 10% FBS, washed in Ca₂₊₋ and Mg₂₊free PBS, and then exposed to various treatments in media containing 1% serum. All treatments were performed in duplicate. The cells were treated with CSE (0.5, 1.0 and 5%) for 1 h at 37°C with 7.5% CO₂. At the end of treatment, the cells were washed with cold, sterile Ca₂₊₋ and Mg₂₊₋ free PBS and were lysed in RIPA buffer as whole lysate (western blotting), and the lysates stored at -80°C.

**Transfection**

The plasmids for dominant negative IKKα and NIK kinase mutant domain on lysine K429 and K430 (K429/A430) were obtained as described previously (2, 42), Transient transfection was performed with 1 µg of plasmids in the presence of Lipofectamine-2000 transfection reagent (product no. 11668-027; Invitrogen, Carlsbad, CA) in MonoMac6- , Ramos B-, and WEHI-231 cells efficiency in case of both plasmids transfection was >80%. Following day after transfection, MonoMac6- and WEHI-231-cells were treated with CSE (0.5%, 1.0% and 2.5%). Whole lysate was used in western blotting analysis. Ramos B cells were pre-treated with 25 µM calpain inhibitor I (ALLN, product no. 208750; Calbiochem, San Diego, CA) for 20 min. The pre-treated cells were washed twice in PBS, and then they were treated with CSE (0.5%, 1.0% and 2.5%) in cells transfected with and without dominant negative IKKα plasmid for 1 h at 37°C with 7.5% CO₂. At the end of treatment, the cells were washed with cold, sterile Ca₂₊₋ and Mg₂₊₋ free PBS and were lysed either in RIPA buffer, and the lysates stored at -80°C.

**Cytoplasmic and nuclear protein extraction**

One hundred milligram of lung tissue was mechanically homogenized in 0.5 ml buffer A [10mM HEPES (pH 7.8), 10 mM KCl , 2 mM MgCl₂, 1 mM DTT, 0.1 M EDTA, 0.2 mM NaF, 0.2 mM Na orthovandate, 1% (vol/vol) NP-40, 0.4 mM phenylmethylsulfonyl fluoride and 1 µg/ml leupeptin] on ice. The homogenate was centrifuged at 2,000 rpm in a benchtop eppendorf centrifuge for 30 s at 4°C to remove cellular debris. The supernatant was then transferred to a 1.7 ml ice-cold micro tube and further centrifuged for 30 s at 13,000 rpm at 4°C. The supernatant was collected as a cytoplasmic extract. The pellet was resuspended in 200 µl of buffer C [50 mM HEPES (pH 7.8), 50 mM KCl, 300 mM NaCl, 0.1 M EDTA, 1 mM DTT, 10% (vol/vol) glycerol, 0.2 mM NaF, 0.2 mM Na orthovandate and 0.6 mM phenylmethylsulfonyl fluoride] and placed on the rotator in the cold room for 30 min. Following centrifugation at 13,000 rpm in a micro eppendorf tube for 5 min, the supernatant was collected as the nuclear extract and kept frozen at - 80°C for western blotting.

**Western blotting**

Lung tissue homogenate samples (cytoplasmic and nuclear proteins) were separated on a 7.5%- 12% SDS-PAGE. MonoMac6, Ramos and WEHI-231 B cells were harvested (24 h posttransfection), and lysed with 10% Igepal CA-630 lysis buffer supplemented with a protease inhibitor cocktail (leupeptin, aprotinin, pepstatin, and PMSF). Equal amount of protein was subjected to electrophoresis on 7.5%-12% PAGE gels, electroblotted onto nitrocellulose membranes (Amersham Bioscience, Piscataway, NJ), and then incubated overnight with primary antibodies at 4°C. The next day, membranes were washed and incubated for 1 h at room temperature with the appropriate secondary antibody linked to horseradish peroxidase (Dako, Santa Barbara, CA), bound complexes were detected with the use of the enhanced chemiluminescence method (Jackson Immunology Research, West Grove, PA).

**Immunoprecipitation**

One hundred milligram of mouse lung tissue homogenate samples were immunoprecipitated with 1 µg of specific antibodies and 20 µ1 of protein A/G agarose beads (product no. SC-2003; Santa Cruz) in RIPA buffer (50 mM Tris-HCl [pH7.4], 150 mM NaCl, 0.25 mM EDTA, 5 mM NaF, 0.1 % sodium deoxycholate, 1% Triton X-100 in PBS) overnight at 4°C. After immunoprecipitation, the precipitates were washed with 10 mM Tris, 1 mM EDTA, 150 mM NaCl, 1mg/ml BSA, 1% Triton X-100 and protease inhibitor in PBS three times with spinning at 2,000 rpm for 1 min at 4°C. The precipitants were resuspended in 50 µl af Laemmli sample buffer to a final concentration of 1 × sample buffer, and they were heated at 95°C for 5 min. The collected supernatants (immunoprecipitants) were run on a 7.5% SDS-PAGE.

**Chromatin immunoprecipitation (ChIP)**

One hundred milligram of lung tissue was homogenized in 1 mg/ml BSA with protease inhibitor cocktail in PBS, and cross-linked with 1% formaldehyde for 10 min, rinsed three times with PBS, and then 0.5 ml of 2.5 M glycine was added. After a brief centrifugation, cell pellets were resuspended in SDS-lysis buffer (50 mM Tris-HCl, 1% SDS, 5 mM EDTA, 5 mM Na-butyrate, protease inhibitors). Sonication of nuclear pellet containing chromatin was performed four times for 30 s and one time for 15 s at a maximum speed using Misonix-3000 Sonicator (Misonix Inc, Farmingdale, NY). Supernatants were collected and diluted (1:10 dilution) with buffer (1% Triton X-100, 2 mM EDTA, 150 mM NaCl, 20 mM Tris-HCl [pH 8.0], 5 mM NaButyrate, protease inhibitor) followed by preclearing the extract with 60 µl of protein A agarose/salmon sperm DNA (Cat no. 16-157, Upstate) for 3 h at 4 °C (43). Immunoprecipitation was carried out overnight at 4 °C with 1 µg of specific antibodies as mentioned above. After immunoprecipitation, 40 µl of protein A agarose/salmon sperm DNA was added and incubated for 2 h and followed by brief centrifugation. Precipitates were washed with Paro buffer I (0.1% SDS, 1% Triton X-100, 2 mM EDTA, 20 mM Tris-HCl pH 8.1, 150 mM NaCl), Paro buffer II (0.1% SDS, 1% Triton X-100, 2 mM EDTA, 20 mM Tris-HCl [pH 8.1], 500 mM NaCl), Paro buffer III (0.25 M LiCl, 1% Igepal CA-630, 1% deoxycholate, 1 mM EDTA, 10 mM Tris-HCl [pH 8.1]) for 5 min at 4°C. Precipitates were then washed again with Tris-buffer twice for 5 min each. The antigen-antibody complexes were extracted two times with 50 µl elution buffer (0.6 µg/µl proteinase K, 1% SDS, 0.1 M NaHCO₃). The eluted samples were heated at 65 °C overnight to reverse formaldehyde cross-linking. The recovered DNA was purified with a QIAquick PCR purification kit (Product no., 28106, Qiagen, Valencia, CA) (43). Samples of input DNA were also prepared in the same way as described above. PCR amplification was performed using a PTC-200 DNA engine (MJ Research, Waltham, MA) under the following conditions: 94 °C for 180 s; 30-38 cycles at 94 °C for 45 s, 60°C for 60 s, and 72 °C for 60 s; and final elongation at 72 °C for 10 min. PCR for the input reaction was performed using 100 ng of genomic DNA. Mouse primer sequences were given in Table 1, and PCR products were analyzed on a 1.5-2.0% agarose gel.

**Table 1: Primer Sequences used in Chromatin Immunaprecipitation assay**

| **Gene** | **Primer** | **Sequence** |
|---|---|---|
| MIP-2 | Sense | 5'- CAA CAG TGT ACT TAC GCA GAC G -3' |
| | Antisense | 5'- CTA GCT GCC TGC CTC ATT CTA C -3' |
| IL-6 | Sense | 5'- GAC ATG CTC AAG TGC TGA GTC AC -3' |
| | Antisense | 5'- AGA TTG CAC AAT GTG ACG TCG -3' |

**Protein assay**

Protein level was measured with a BCA kit (Pierce, Rockford, IL). Protein standards were obtained by diluting a stock solution of BSA. Linear regression was used to determine the actual protein concentration of the samples.

Statistical analysis

Results are shown as means ± SEM. Statistical analysis of significance was calculated by oneway ANOVA followed by Fisher's PLSD post-hoc test for multigroup comparisons (StatView 5.0, SAS Institute, Cary, NC). Statistical significance is indicated in figure legends.

**Example 2 - CS exposure increased the levels of RelB and RelA/p65 in alveolar/airway epithelial cells in mouse lung.**

The xpression and localization of RelB and RelA/p65 in mouse lung sections were studied by immunostaining of RelB in mid-sagittal sections in response to CS exposure. RelB and RelA/p65 positive cells with increased staining of RelB were detected in macrophages, type II alveolar and airway epithelial cells in mouse lung tissue exposed to CS **(****Figures 1A and 1B****).** However, B-cells were unable to be detected using selective cell surface marker CD19+ expression in lung sections of mouse exposed to CS for acute (3-days) exposure **(****Figure 1A****)** but CD 19+ cells were increased in mouse lung after 8-weeks of CS exposure compared to air-exposure (data not shown). These observations suggest that CS caused the increased levels of RelB in mouse lungs in response to acute CS exposure.

**Example 3 - CS exposure increased the levels of RelB and its interaction with NIK and p52 in mouse lung.**

The levels of RelB and its interactions with NIK and p52 were determined in lung tissue of mice exposed to CS for 3 days. The levels of RelB and NIK were significantly increased in both nucleus and cytoplasm of mouse lung tissue, whereas the level of p52 was increased only in cytoplasm but not in the nucleus in response to CS **(****Figures 2A and 2B****).** The interaction of RelB with NIK and p52 in mouse whole lung tissue was also determined by immunoprecipitation with relevant antibodies. CS increased the level of RelB interaction with both NIK and p52 in mouse lungs **(****Figure 2B****).** These data showed that CS exposure increased the levels of both RelB and NIK in the nucleus as well as increased the interactions of RelB with NIK and p52 in mouse lung.

**Example 4- CS exposure caused recruitment of RelB on pro-inflammatory gene promoters in mouse lung.**

It has been shown that increased level of RelB is pre-requisite for alternate pathway of NF-κB dependent gene transcription in response to various pro-inflammatory stimuli (20, 21, 23, 27, 44, 45). Therefore, it was hypothesized that CS exposure induces the pro-inflammatory mediators by recruiting RelB on the promoters of pro-inflammatory genes. To test this hypothesis, chromatin immunoprecipitation (ChIP) assay was performed using the antibodies against RelB in lungs of mice exposed to CS. As expected, the levels of RelB on the pro-inflammatory promoter sites of MIP-2 and IL-6 were increased **(****Figures 3A and B****).** However, the regions which were noncoding for IL-6 and MIP-2 showed no change in gene expression validating the specificity of the ChIP assays (Yang *et al,* data not shown). These results demonstrated that RelB is recruited on the promoters of various pro-inflammatory genes thereby exerting its effect on pro-inflammatory gene expression in mouse lung.

**Example 5 - CS increased the levels of NF-κB-dependent pro-inflammatory mediators in mouse lung.**

It was next determined whether CS induces the levels of RelB-dependent pro-inflammatory cytokines in mouse BAL fluid measured by Luminex-based multiplex assay. The proinflammatory mediators, such as CD40, CD40 ligand (which are present on antigen-presenting cells and are costimulatory molecule for proliferation and enhanced survival of T cells), eotaxin and granulocyte chemotactic protein-2 (GCP-2) which are thought to be regulated by alternative NF-κB pathways were significantly increased in BAL fluid in response to CS exposure (Figure 4). Previously the present inventors have shown that the levels of MIP-2 and IL-6 were increased in BAL fluid at 3 days of CS exposure (2). Taken together, these data suggested that CS induces the alternative pathway of NF-κB-dependent pro-inflammatory mediators in mouse lung.

**Example 6 - CSE increased the levels of RelB, and IKKα is a critical regulator of RelB in monocyte/macrophages (MonoMac6 cells).**

Macrophages are known to play an important role in abnormal inflammatory response seen in subjects with COPD and recently the present inventors have shown that CS induces the levels of pro-inflammatory mediators by NF-κB-dependent mechanism in macrophages (2, 4). We, therefore, determined whether RelB is also activated in response to CSE in monocyte/macrophages (MonoMac6 cells) leading to RelB-dependent pro-inflammatory cytokines release. Similar to the activation of RelA/p65 (2), it was found that RelB is activated in response to CSE treatments associated with increased levels of its partner p52 in these cells **(****Figures 5A and 5B****).** These data suggested that CS activates both the classical- and the alternative-NF-κB pathways in macrophages leading to pro-inflammatory mediators release. It has been recently shown that NIK activates IKKα homodimer in IKKs complex which in turn resulted in activation of alternative NF-κB pathway characterized by nuclear translocation of RelB (22, 44). Therefore, it was determined whether NIK and IKKα are involved in regulation of RelB in response to CSE in MonoMac6 cells. Macrophages were transfected with dominant negative IKKα or double mutant of NIK (K429/A430), and treated with CSE (0.5%. 1.0% and 2.5%) for 1 hour. The levels of RelB and p52 were increased in response to CSE in nontransfected and untreated cells, whereas the levels of RelB was attenuated in both dominant negative IKKα- and double mutant of NIK (K429/A430, data not shown)- transfected MonoMac6 cells. Transfection of wild-type IKKα increased the levels of RelB and p52 suggesting that IKKα and NIK are required for CS-mediated activation of alternative NF-κB pathway in macrophages **(****Figure 5A and 5B****).**

**Example 7 - CSE rapidly degraded RelB, and the loss of IKKα partially restored RelB in B-cells (human Ramos B-cells and mouse WEHI-231 B-cells).**

It has been shown that immune-inflammatory lymphoid cells are important in the pathogenesis of COPD (12, 13), and it is known that RelB controls the alternate NF-κB pathways in these cells by forming a transcriptionally inactive complex with RelA/p65 (20, 21, 24, 25, 46). Hence, the levels of RelB in response to CSE in lymphoid B-cells were determined (human Ramos B-cells and mouse WEHI-231 B-cells). In contrast to RelA/p65 activation in macrophages, it was found that RelB is rapidly degraded in response to CSE treatments in these cells **(****Figures 6A and 6B****).** It was next determined whether CS-mediated degradation of RelB is associated with down modulation of its signaling by NIK and IKKα in these cells. Surprisingly, it was found that CSE caused activation of both NIK and IKKα, and transient transfection with dominant negative IKKα partially whereas transfection of double mutant of NIK (K429/A430) completely restored CSE-mediated loss of RelB in B-cells **(****Figures 6** **and** 7). However, the levels of NIK and RelA/p65 were not changed in response to CSE in cells transfected with dominant negative IKKα plasmid compared to non-transfected cells. Furthermore, CSE-mediated reduced level of RelB was partially attenuated whereas the levels of NIK and RelA/p65 were normalized in cells transfected with wild-type IKKα. These data suggested that IKKα activation may not be critical for CSE-mediated loss of RelB in B-cells.

**Discussion of RelB Results**

It has been shown that the absolute volume of infiltrated inflammatory immune cells in lymphoid follicles in the small airways is associated with the progression of COPD (12), suggesting that recruitment of lymphoid cells (T- and B-lymphocytes) in the lung may occur in response to chronic CS exposure. This concept is supported by a recent study showing that B-cells are increased in lungs of subjects with emphysema as well as in mouse lungs in response to chronic CS exposure (13). These studies suggest that CS recruits B-cells into the lung, however, the functional role of B-cells in pathogenesis of COPD is not known.

In this study, the alternative NF-κB signaling events that control the activation of RelB in B-lymphocytes (B-cells) and macrophages *in vitro* and in mouse lung *in vivo* in response to CS exposure were studied. It was shown that CS differently regulate the level of RelB in lung structural cells, macrophages and B lymphocytes, suggesting a dual and opposing effects of CS on RelB activation in cell specific manner. It is known that RelB activation is tissue- and cell-specific, especially in lymphoid organs and lymphoid cells, such as B and T-cells, and fibroblasts (20-22, 24, 2S). In these cells, RelB acts as an inhibitor of transcription of various pro-inflammatory genes whereas it functions as pro-inflammatory in non-lymphoid cells (47). Here this knowledge was extended to study the expression of RelB in lung structural cells, airway/alveolar epithelial cells and alveolar macrophages in response to CS. Immunohistochemical staining of mouse lung tissue sections demonstrated the localization of RelB in airway and alveolar epithelial cells, as well as in alveolar macrophages in response to CS exposure. Similarly, our data show increased levels of RelB in lungs of mouse exposed to CS. These data corroborate with recent studies showing that TNF-α stimulation resulted in strong increase in levels of RelB in both the cytoplasm and the nucleus of mouse intestinal cells and macrophages as well as in various lymphoid cells (48, 49). Furthermore, TNF-α is induced in response to CS exposure in mouse lung (2). Hence, CSEinduced TNF-α release may activate RelB pathways in mouse lung.

RelB-containing complexes are shown to act as both activators and repressors of NF-κBdependent gene transcription (25). For instances, the recruitment of RelB to the IL-12p40 promoter correlates with transcriptional down-regulation, whereas RelB upregulates gene expression of a variety of pro-inflammatory mediators, such as CD40, CD40 ligand, eotaxin, GCP-2, ELC/CCL19 (EBII ligand chemokine), MDC (macrophage-derived chemokine), RANTES (regulated upon activation, normal T-cell expressed and secreted), MIP-1α, MIP-1β,MIP-2, IP-10, MCP-1, KC/CINC (IL-8), IL-13, IL-1β, TNF-α and IL-4 genes (13, 21, 50, 51).

The present inventors have previously shown that CSE causes activation of various pro-inflammatory cytokines in macrophages (MonoMac6 cells) and airway epithelial cells (2, 4, 41). Here it is shown that RelB is recruited on the promoters of pro-inflammatory cytokine genes in mouse lung tissues by CS exposure suggesting that these cytokines are upregulated due to RelB and RelA/p65 activation in macrophages and epithelial cells. It was observed that RelB was also activated in monocyte/macrophages (MonoMac6 cells) exposed to CSE. This was associated with increased activation of p52 which forms active RelB:p52 complex. CS increased the level of RelB associated with its interaction with p52 and NIK in mouse lung suggesting that this complex is active for gene transcription which is confirmed by the ChIP assay. It is known that p100 is the main inhibitor of RelB and generation of p52/RelB results from proteolytic cleavage of a unique pool of p100/RelB (44). p100 functions as IκBα inhibiting the RelB-mediated gene transcription. Furthermore, p100 is directly phosphorylated by IKKα-NIK and cause its processing into p52 in the cytoplasm (27,44). RelB:p100 complex is inhibitory whereas RelB or RelB:p52 cause induction of pro-inflammatory genes. Our observation of increased levels of p52 in the cytoplasm implies that p100 is cleaved by IKKα signaling in mouse lungs in response to CS. However, the level of p52 in the nucleus was not detected suggesting that RelB directly binds on the promoters of pro-inflammatory genes as shown by the ChIP assay.

It is interesting to note that RelB is differentially regulated by CS in mouse lung tissue, macrophages and B lymphocytes. Surprisingly, RelB is rapidly degraded in B-cells in response to CSE treatments. The question is asked regarding the signaling mechanism whereby RelB is regulated in these cells, and what is the significance of this opposing effect? Numerous experimental data indicated that NIK and IKKα act as an activator of RelB:p52-NF-κBcontrolled gene transcription and lymphoid cells proliferation (52, 53). Furthermore, it is known that IKKα regulates the late differentiation of B-cells by intrinsic NIK-IKKα signaling (22). Our data show that RelB is associated with NIK in CS-exposed mouse lung tissue. It was further determined whether NIK and IKKα are involved in regulation of RelB in response to CSE in MonoMac6 cells. CSE-induced levels of RelB was reduced when the cells were transfected with dominant negative IKKα or double mutant of NIK (K429/A430) whereas transfection of wildtype IKKα increased the levels of RelB suggesting that NIK-IKKα signaling is required for CSmediated activation of RelB in macrophages. The similar approach of gain and loss of NIK and IKKα was then used and it was determined whether CS-mediated degradation of RelB is associated with down modulation of its signaling by NIK and IKKα in B-cells. Surprisingly, it was found that CSE caused activation of both NIK and IKKα, and transient transfection with dominant negative IKKα or double mutant of NIK (K429/A430) partially restored CSE-mediated loss of RelB in B21 cells. These data suggested that apart from NIK-IKKαactivation there is another signaling mechanism whereby for CSE-mediated regulation of RelB in B-cells. On the contrary to lung cells and macrophages, the level of RelB was decreased in B-cells suggesting that its degradation is regulated by proteasome-dependent mechanism.

It is known that RelB is degraded by rapid phosphorylation at amino acids Thr84 and Ser52 followed by cleaving N-terminal amino acids and complete degradation in the proteasomes (24). Our data show that the proteasome inhibitor (ALLN) prevented the degradation of RelB in response to CSE in B-cells. These results agree with the notion that RelB is an essential regulator required for suppression of NF-κB function and modulation of chemokine expression in activated B- and T-cells, and fibroblasts (21). This contention is supported by the observations that the disruption of the RelB locus resulted in impaired cellular immunity and severe pathology associated with dysfunction of the hematopoietic system and inflammatory response in lungs of RelB-/-mice (54). Furthermore, Xia and co-workers has also demonstrated that RelB is an important regulator of chemokine expression in mouse fibroblast and lymphoid cells, thereby playing a key role in the resolution of acute inflammation by inhibiting RelA/p65 (21).RelB is known to dampen RelA/p65 activity (20, 25). RelB forms transcriptonally inactive complexes with RelA/p65 so that RelA/p65 is unable to find to κB-sites in fibroblasts (25). Moreover, serine-276 domain of RelA/p65 seems to be a critical phosphorylation site for TNF-α- induced RelA/RelB complex formation. It is possible that RelB degradation would lead to RelA/p65 activation as seen in lymphoid cells in response to CSE. It has been shown that overexpression of RelB suppressed the LPS-induced NF-κB activity and pro-inflammatory mediators release in fibroblasts (20). It is also known that RelB modulate IκBα stability and suppresses

**Example 8 - Materials and Methods - SIRT1 Experiments**

**Materials**

Unless otherwise stated, all reagents used in this study were purchased from Sigma (St. Louis, MO). Penicillin, streptomycin, and RPMI 1640 were obtained from Life Technologies (Geithersburg, MD). Rabbit polyclonal anti-SIRT1 (Ab7343) and mouse monoclonal anti-SIRT1 (05-707) antibodies were procured form Abcam (Cambridge, MA) and Upstate (Lake Placid, NY), respectively. Antibodies against NF-κB-RelA/p65 (rabbit polyclonal; sc-372), lamin B (goat polyclonal; sc-6216), and α-tubulin (mouse monoclonal; sc-5286) were purchased from Santa Cruz Biotechnology (Santa Cruz, CA). Mouse monoclonal antibodies against 4-hydroxy-2-nonenal (24327), nitrotyrosine (05-233) and β-actin (CP01) were obtained from Oxis International (Foster City, CA), Upstate (Lake Placid, NY), and Calbiochem (La Jolla, CA), respectively. Human SIRT1 siRNA (L-003540-00), non-target scrambled siRNA (D-001810-01) and DharmaFECT2 transfection reagent (T-2002-01) were purchased from Dharmacon (Lafayett, CO, USA). The anti-acetylated RelA/p65 (AcK310) antibody (33) was provided by Dr. Leonard Buckbinder at Pfizer Global R&D (Groton, CT). Overexpression SIRT1 plasmid and SIRT1 construct lacking deacetylase domain (SIRT1-H363Y) were obtained from Addgene (Cambridge, MA; www.addgene.org).

**Collection of human lung tissues**

Lung tissue specimens from 37 subjects/subjects including 10 life-long non-smokers, 10 current smokers with normal lung function, and 9 subjects with COPD (3 former- and 6 current-smokers; 2 subjects had been prescribed inhaled steroids) undergoing resection for suspected lung carcinoma /FEVC <70% and bronchodilatation effect <12 % (55). None of the subjects had suffered from acute exacerbation for two months. Tumor-free peripheral lung tissues were immediately stored at -80°C for Western blot analysis and preserved for immunohistochemistry as described by Dail and Hammar (81). The clinical characteristics of the subjects are shown in Table 2. lung tumor (either malignant or non-malignant-local carcinoma or hamartoma), and 8 subjects (former-smokers; all had been prescribed inhaled and/or low dosage oral corticosteroids) with severe COPD undergoing lung transplantation were collected (both COPD groups were pooled; see table 2) from the Departments of Medicine and Pathology, Helsinki University Hospital. COPD was defined according to GOLD criteria (FEV₁ <80% of predicted, FEV₁

**Table 2 A summary of the clinical characteristics of objects and patients**

| | **Non-smokers** | **Smokers** | **COPD** |
|---|---|---|---|
| Number(n) | 10 | 10 | 17 |
| M:F ratio | 6:4 | 8:2 | 9:8 |
| Age, years | 59±15 | 58±4 | 60±7 |
| Smoking, Pack Years | 0 | 34±18*** | 42±19*** |
| FEV₁% predicted | 98 ± 4 | 97 ± 13 | 39 ± 27^{†††} |
| FEV₁/FVC (%) | 81 ± 3 | 81 ± 2 | 51 ± 23* |
| DCO % pred | 80 ± 12 | 90 ± 10 | 54 ± 23* |
| DCO/VA % pred | 86 ± 14 | 103 ± 13 | 70 ± 24 |

| | | | |
|---|---|---|---|
| *Definition of abbreviations:* COPD = Chronic obstructive pulmonary disease, M:F ratio = Male:Female ratio, FEV₁ = Forced expiratory volume in one second, FVC = Forced vital capacity, DLCO = Diffusing capacity of lung for carbon monoxide, VA = Alveolar volume, Data shown represents mean ± SEM, *p<0.05 compared with non-smokers and smokers without COPD; ***p<0.001 compared with non-smokers; ^{†††}p<0.001 compared with non-smokers and smokers without COPD. | | | |

**MonoMac6 cell culture**

The human monocyte-macrophage cell line (mature monocytes) MonoMac6, which was established from peripheral blood of a subject with monoblastic leukemia (82, 83), were grown in RPMI 1640 medium supplemented with 10% FBS, 2 mM L-glutamine, 100 µg/ml penicillin, 100 U/ml streptomycin, 1% nonessential amino acids, 1 mM sodium pyruvate, 1 µg/ml human holo-transferrin, 8 µg/ml polymixin B, 9 µg/ml bovine insulin, and 1 mM oxaloacetic acid. The cells were cultured at 37 °C in a humidified atmosphere containing 7.5% CO₂.

**Preparation of cigarette smoke extract (CSE)**

CSE (10%) was prepared by bubbling smoke from one research grade cigarette (1R3F; University of Kentucky, Lexington, KY) into 10 ml of RPMI 1640 medium with 1% FBS, as described previously (21, 30, 31).

***In vitro* treatments**

MonoMac6 cells were seeded at a density of 1.5 x 10⁶ cells/well in six-well plates containing 2 ml of culture medium supplemented with 1% FBS, starved for overnight, and then treated with different concentrations of CSE (0.1, 0.5, and 1.0%) at 37 °C. After 1, 4 or 24 hr of treatment, the cells and culture media were harvested.

**Experimental procedures**

The detailed procedures for western blotting, immunoprecipitation, immunohistochemistry, immunocytochemistry, mRNA expression and siRNA or cDNA transfection are provided as follows.

**Preparation of cigarette smoke extract (CSE)**

Research grade cigarettes (1R3F) were obtained from the Kentucky Tobacco Research and Development Center at the University of Kentucky (Lexington, KY). The composition of 1R3F/cigarettes was: total particulate matter: 17.1 mg, tar: 15 mg, and nicotine: 1.16 mg. CSE (10%) was prepared by bubbling smoke from one cigarette into 10 ml of culture media supplemented with 1% FBS at a rate of one cigarette/ 2 minutes as described previously (101,102), using a modification of the method described earlier by Carp and Janoff (103). The pH of the CSE was adjusted to 7.4, and was sterile filtered through a 0.45 µm filter (25 mm Acrodisc; Pall Corporation, Ann Arbor, MI). CSE preparation was standardized by measuring the absorbance (OD 0.72 ± 0.02) at a wavelength of 320 nm. The pattern of absorbance (spectrogram) observed at λ₃₂₀ showed a very little variation between different preparations of CSE. CSE was freshly prepared for each experiment and diluted with culture media supplemented with 1% FBS immediately before use.

**Extraction of nuclear protein**

MonoMac6 cells or human lung homogenates were washed with ice-cold PBS, resuspended/homogenized in buffer A (10 mM HEPES, pH 7.9, 10 mM KCl, 0.1 mM EDTA, 0.1 mM EGTA, 1 mM DTT, and 0.5 mM PMSF) and allowed to swell on ice for 15 min. 10% Nonidet P-40 was added to the tubes, vigorously vortexed for 15 sec and centrifuged to collect the supernatant containing cytosolic proteins. The pelleted nuclei were resuspended in buffer B (20 mM HEPES, pH 7.9, 0.4 M NaCl, 1 mM EDTA, 1 mM EGTA, 1 mM DTT, and 1 mM PMSF) and kept on ice for 30 min. After vortex for 20 sec, the cell lysates were centrifuged, and supernatants containing the nuclear proteins were collected.

**Western blot analysis**

Equal amount of (30 µg) nuclear proteins from each group were resolved by electrophoresis on 7.5% sodium dodecyl sulfate polyacrylamide (SDS-PAGE) gels and electro-blotted onto nitrocellulose membrane (Amersham, Arlington Heights, IL). The nitrocellulose membrane was blocked with 5% nonfat dry milk for 1 hr at room temperature, and incubated with the primary antibody at 4°C for overnight (1:1,000 dilutions in 5% BSA). After being washed with phosphate-buffered saline containing 0.05% TWEEN-20, the membrane incubated with respective secondary antibody (1:10,000 dilution in 5% BSA for 1 hr at room temperature) linked to horseradish peroxidase (Dako, Santa Barbara, CA, USA). Proteins were detected by enhanced chemiluminescence method (Jackson Immunology Research, West Grove, PA), and were quantified using the image processing and analysis software, ImageJ (NIH software). Protein levels were expressed as percent of controls. Levels of the housekeeping protein β-actin were used for normalization.

**Immunohistochemistry**

Buffered formalin (10%) fixed paraffin embedded lung sections (3-µm thick) of non-smokers, smokers and COPD patients were deparaffinized using xylene and rehydrated in a graded ethanol series. Heat-induced antigen retrieval was performed in a microwave oven before immunohistochemical staining. After cooling and in running tap water, endogenous peroxidase activity was blocked by incubating in 3% hydrogen peroxide. To avoid the non-specific background, blocking was done with 5% BSA-PBS solution for 1 hr at room temperature. For the detection of SIRT1 protein, the slides were incubated with polyclonal rabbit anti-SIRT1 (1:100 dilution) at 4°C for over night in a humidified chamber. The signal conversion was carried out with avidin-biotin-peroxidase complex (ABC) method, as described by Toyokuni (104) followed by hematoxylin counter staining. The assessment of immunostaining intensity was performed semi-quantitatively and in a blinded fashion. For the detection of NF-κB RelA/p65 protein, the slides were incubated with rabbit polyclonal anti-RelA/p65 (1:100 dilution) at 4°C for over night in a humidified chamber. Subsequent incubations with FITC conjugated anti-rabbit secondary antibody for 20 min in dark, the slides were rinsed in PBS and mounted with Vectashield mounting medium (Vector Laboratories, Inc., Burlingame, CA). Labeled tissues were viewed and photographed with a Nikon Eclipse TE2000-S phase-contrast microscope with fluorescence optics and a Nikon COOLPIX 5400 camera.

**Immunocytochemistry**

MonoMac6 cells were treated with CSE and washed with ice-cold phosphate buffered saline and fixed with 4% paraformaldehyde in PBS. To detect the nuclear protein (SIRT1), the cells were permeabilized with 0.1% Triton X-100 and blocked with 10% goat serum for 1 hr at room temperature. The immunostaining was performed using polyclonal rabbit anti-SIRT1 followed by the avidin-biotin-peroxidase complex (ABC) method and counterstained with hematoxylin, as described by Toyokuni (104).

**Immunoprecipitation and immunoblotting**

After the extraction of nuclear proteins from MonoMac6 cells and human lung homogenates, SIRT1 antibody (1:80 dilution; Abcam) was added to 100 µg of nuclear protein in a final volume of 400 µl of RIPA buffer and incubated for 1 hr. Protein-A/G agarose beads (20 µl) (Santa Cruz) were added to each sample and left overnight at 4°C on a rocker. The samples were then centrifuged at 13,000 rpm at 4°C for 5 min. The supernatant was discarded, and the beads were washed three times and then resuspended in 40 µl of lysis buffer. For Western blots, 100 µg of the immunoprecipitated SIRT1 agarose bead suspension were added to 10 µl of 5x sample buffer, boiled, and resolved by SDS-PAGE as described above. To determine the post-translational modification of SIRT1, blots were probed with anti-4-hydroxy-2-nonenal antibody, stripped, and reprobed with anti-3-nitrotyrosine antibody.

**Reverse transcriptase polymerase chain reaction**

After treatments, total RNA was isolated from MonoMac6 cells using RNeasy kit (Qiagen, Valencia, CA, USA). Reverse transcriptase-polymerase chain reaction (RT-PCR) was performed using oligo(dT) primers and superscript reverse transcriptase (Invitrogen Life Sciences) following the manufacturer's recommendations. The PCR conditions for the house keeping gene GAPDH were 20 thermal cycles of 94°C for 45 s, 60°C for 45 s, and 72°C for 90 s, followed by final extension for 10 min at 72°C. SIRT1 was subjected to 35 thermal cycles of 95°C for 30s, 55°C for 30s, 72°C for 30 sec followed by an extension at 72°C for 10 min. The primer pairs were as follows (forward and reverse, respectively): hSIRT1 (Integrated DNA technologies (IDT), IA, USA., 5'-TCA GTG TCA TGG TTC CTT TGC-3' and Up: Rev: 5'-AAT CTG CTC CTT TGC CAC TCT-3' (Product size 200 bp), and GAPDH, 5'-AGTGTAGCCCAGGATGCCCTT-3' and 5'-GCCAAGGTCATCCATGACAAC-3'. Amplified products were resolved by 1.5% agarose gel electrophoresis, stained with ethidium bromide, visualized and scanned by a white/UV transilluminator and quantified by densitometry.

**Transfection of siRNA**

Predesigned SIRT1 siRNA duplex (sense sequence: GAUUGGGUACCGAGAUAUU, antisense sequence: 5'-PAAAGUAUAUGGACCUAUCCUU), which is not homologous to other isoforms, was used to knock-down human SIRT1. siCONTROL non-targeting scrambled siRNA (5'-UAGCGACUAAACACAUCAAUU-3') was used as a negative control. Human MonoMac6 cells were transfected with SIRT1 siRNA (L-003540-00) or non-target scrambled siRNA (D-001810-01) using DharmaFECT2 transfection reagent (T-2002-01) according to manufacturer's (Dharmacon, Lafayette, CO, USA) instructions. Briefly, 100 nM siRNA was mixed with the transfection reagent and incubated for 20 min at room temperature. The mixture was added to the 0.2 X 10⁶ cells in the 12-well plate and incubated at 37 °C. At 36-48 hr after transfection, the cells were washed and used for the treatments.

### Transfection of SIRT1 and SIRT1-H363Y

MonoMac6 cells were transfected with SIRT1 and SIRT1 deacetylase defective mutant or deacetylase lacking mutant-SIRT1-H363Y plasmids (both obtained from Addgene, Cambridge, MA) using the commercially available calcium phosphate transfection kit (Invitrogen, Carlsbad, CA) according to the manufacturer's instructions. Briefly, MonoMac6 cells were seeded at 0.2 X 10⁶ cells/well in 12-well plate and were transfected with 20 µg of SIRT1 and SIRT1-H363Y constructs using the calcium phosphate transfection method. Two days after transfection, cells were incubated in a absence or presence of CSE (0.5%) for 4 h. The cell free culture medium was collected at the end of the experiment for IL-8 assay.

**Statistical analysis**

Statistical analysis of significance was calculated by one-way ANOVA followed by Tukey's post hoc test, using StatView software. Results are shown as mean ± SEM of at least three independent experiments.

**Ethical considerations**

The study was approved by the ethical committee of Helsinki University Hospital District. The volunteers gave their written informed consent.

**Example 9 - Decreased levels of nuclear sirtuin (SIRT1) in peripheral lung tissues of smokers and subjects with COPD were associated with increased post-translational modifications by reactive aldehydes and nitric oxide products**

To determine the levels of SIRT1, the peripheral lung samples were collected from non-smokers, smokers and subjects with COPD. Levels of nuclear SIRT1 were measured by western blotting and normalized with the amount of β-action (loading control). The levels were significantly lower (p<0.001) in nuclear extracts of peripheral lung tissues of smokers and subjects with COPD than in the lungs of non-smokers (Fig. 9A, B). The reduction in the levels of SIRT1 was more pronounced in subjects with COPD compared to that of smokers. Similar reduction in SIRT1 activity was also observed in nuclear extracts (data not shown). In view of the presence of pro-oxidative and nitrosative components in CS and our observation of decreased SIRT1 levels in response to CSE (see above), it was investigated whether oxidative/nitrosative protein modifications are involved in the observed reduction in SIRT1 levels. Post-translational/covalent modification of SIRT1 proteins was assessed by immunoprecipitation, followed by Western blot analysis using monoclonal antibodies for 4-hydroxy-2-nonenal (4-HNE) and 3-nitrotyrosine. There was a significant increase in tyrosine nitration and 4-HNE (carbonyl adducts) modification on SIRT1 in lungs of smokers and subjects with COPD compared to non-smokers (Fig 19C, D).

Immunohistochemical staining of fixed peripheral lung tissues confirmed the decrease in the levels of SIRT1 in macrophages and airway/alveolar epithelial cells both in the lungs of smokers and in subjects with COPD when compared to non-smokers (Fig. 10A, B). These data suggest that the level of SIRT1 is decreased by oxidative and/or pro-inflammatory effects of CS both in smokers and in subjects with COPD.

**Example 10 - Decreased level of SIRT1 was associated with increased activation of RelA/p65 NF-κB in the lungs of smokers and subjects with COPD**

To determine whether decreased levels of SIRT1 is associated with the expression of NF-κB-dependent pro-inflammatory cytokines, the level of RelA/p65 subunit of NF-κB was assessed in the peripheral lung tissues of smokers and subjects with COPD, and compared with non-smokers. The expression of NF-κB was increased in lung macrophages and epithelial cells of smokers and subjects with COPD as compared to non-smokers (Fig. 11A, B) suggesting that SIRT1 reduction is associated with NF-κB activation.

**Example 11- SIRT1 protein levels and mRNA expression were reduced by cigarette smoke extract (CSE) treatment in MonoMac6 cells**

To determine the molecular regulation of decreased SIRT1 levels in lungs (predominantly in macrophages) of smokers and COPD subjects, the effect of CSE on SIRT1 levels was investigated in human monocyte-macrophage cell line (MonoMac6). CSE (0.1, 0.5, and 1.0%) decreased nuclear SIRT1 protein levels at 4 and 24 hr of treatments (Fig. 12A, B). Similar reduction in SIRT1 activity was also observed in response to CSE treatments in MonoMac6 cells (data not shown). To further characterize the mechanism of CS-mediated decrease in SIRT1 levels, the level of SIRT1 mRNA in CSE-treated MonoMac6 cells was examined using qualitative reverse transcriptase-polymerase chain reaction (RT-PCR). CSE produced marked decreases in SIRT1 mRNA levels in macrophages at 24 h but not at 4h (Figure 12C).

Immunocytochemical staining for SIRT1 confirmed the localization of SIRT1 in the nucleus of MonoMac6 cells, which were dramatically decreased in response to CSE treatments (Fig. 13A,B). This finding suggests that CS-mediated modification of SIRT1 occurs at earlier time points (4 h), which leads to its degradation whereas the reduction of mRNA level occurs at later time points (24 h). This finding corroborates with our results that CSE decreased SIRT1 protein levels in MonoMac6 cells at 4 h. Thus, it appears that decrease in SIRT1 levels/enzyme activity due to CS (post-translational effect) might precede the transcriptional effects (see below).

**Example 11 - CSE induced IL-8 release in MonoMac6 cells**

To determine the effect of CS on pro-inflammatory cytokine release *in vitro,* MonoMac6 cells were exposed to different concentrations of CSE. Culture media was collected to assay the IL-8 release by ELISA. CSE (0.1, 0.5, and 1.0%) significantly increased IL-8 release from these cells at 4 (p<0.01 or p<0.001) and 24 hrs (p<0.001) of treatments (Fig. 14). Assay of lactate dehydrogenase (LDH) leakage showed no cytotoxicity of CSE (0.1 - 1.0%) in MonoMac6 cells at 4 hr and 24 hr. These results confirmed the pro-inflammatory effect of CS, and the increased release of pro-inflammatory cytokines was associated with decreased levels of SIRT1 in MonoMac6 cells.

**Example 12 - SIRT1 deacetylase regulates IL-8 release from MonoMac6 cells in response to CSE**

To characterize the role of SIRT1 in CS-mediated IL-8 release, the endogenous SIRT1 was knocked down, SIRT1 was over-expressed, or the catalytic mutant of SIRT1 (H363Y, SIRTI lacking deacetylase domain) was over-expressed in MonoMac6 cells, which were then treated with CSE (0.5%) for 4 or 12 h. SIRT1 levels were decreased by SIRT1 siRNA transfection (~60%, data not shown; without any change in SIRT2 or SIRT3 levels) and/or CSE treatment. Knock-down of SIRT1 or CSE treatment resulted in significantly (p<0.001) increased IL-8 release in MonoMac6 cells as compared to control and non-scrambled siRNA transfected group. There was a further increase in the level of IL-8 release in CSE-treated SIRT1 siRNA-transfected cells as compared to CSE-treated cells alone (Fig. 15A). Over-expression of neither SIRT1 nor H363Y showed any significant change in IL-8 release at 4 hr, but in response to CSE treatment overexpression of SIRT1 decreased the IL-8 release, whereas H363Y increased the IL-8 release as compared with CSE treated group (Fig. 15B). Taken together, these data show a negative correlation in the levels/deacetylase activity of SIRT1 and IL-8 release, further confirming the involvement of SIRT1 in CS-mediated IL-8 release.

**Example 13 - SIRT1 is post-translationally modified by CSE-derived reactive oxygen/nitrogen species and reactive aldehydes in MonoMac6 cells**

Based on our data of post-translational modifications of SIRT1 by reactive aldehydes and nitric oxide in lungs of smokers and subjects with COPD, it was determined whether CSE is directly involved in such modifications by its reactive components in MonoMac6 cells. Indeed, our data show post-translational modification of SIRT1 protein by 4-HNE and 3-nitrotyrosine in response to CSE in MonoMac6 cells confirming the direct involvement of CS-components, rather than inflammatory cell ROS production, in covalent modification of SIRT1 (Fig. 16A,B).

**Example 14 - CS-mediated reduction of SIRT1 was associated with increased acetylation and activation of RelA/p65 NF-κB**

To examine the relationship between CSE-mediated acetylation/activation of NF-κB and IL-8 release, MonoMac6 cells were treated with CSE (0.5 and 1.0%) and analyzed for acetylated RelA/p65 using a specific acetylated lysine 310 residue (K310) antibody for RelA/p65. Acetylation of RelA/p65 at lysine310 residue is critical for sustained pro-inflammatory cytokine release (86, 87). Western blot analysis revealed that CSE significantly increased the acetylation and thereby activation of RelA/p65 (Fig. 17A, B). CSE treatment decreased the SIRT1 deacetylase level (Fig. 12, 13), which was associated with increased acetylation of RelA/p65 levels in the nucleus of MonoMac6 cells.

**Example 15 - Depletion of SIRT1 leads to augmentation of CSE-mediated acetylation of RelA/p65 NF-κB**

Further experiments were performed to determine whether SIRT1 regulates acetylation/activation of NF-κB, since it is known that SIRT1 interacts with RelA/p65 NF-κB (75), and the depletion of SIRT1 was associated with increased expression of ReIA/p65 NF-κB in the peripheral lungs of smokers and subjects with COPD (Fig. 11). SIRT1 knock down alone significantly (p<0.001) increased the acetylation RelA/p65 NF-κB in MonoMac6 cells. In combination with CSE treatment, SIRT1 knock down augmented the acetylating effect of CSE on RelA/p65 (Fig. 18 A,B). This suggested that SIRT1 regulates the acetylation and activation of RelA/p65 NF-κB (lysine 310 residue) in the nucleus.

**DISCUSSION - SIRT1 Experiments**

Recent focus on COPD research is to understand the factors or molecular mechanisms involved in underlying pathogenesis of lung inflammation in smokers who are susceptible to chronic obstructive pulmonary disease (COPD). The present inventors have previously shown that pro-inflammatory cytokine release was increased in human monocytic-macrophage cell line (MonoMac6) *in vitro* and in rat lung *in* vivo in response to CS exposure (88). This was associated with increased NF-κB activation and acetylation of histone proteins (79, 85). However, the molecular mechanisms of CS-mediated lung inflammation particularly in subjects with COPD are not completely understood. This study was focused on the role of class III deacetylases, sirtuin (SIRT1) which is an important protein involved in deacetylation of proteins/histones, regulation of pro-inflammatory cytokine release, apoptosis, stress resistance, metabolism, senescence, differentiation and aging (73, 75, 89, 90). All of which are linked to the pathogenesis of COPD (58-60, 62, 78). The levels of SIRT1 were decreased in peripheral lungs particularly in alveolar macrophages, airway epithelium, and in alveolar epithelium of smokers and subjects with COPD as compared with non-smokers. Since SIRT1 is involved in the regulation of NF-κB (73, 75), the decreased levels of SIRT1 may result in NF-κB-mediated abnormal chronic inflammatory effect which is observed in lungs of smokers and in subjects with COPD. Consistent with this notion, decreased levels of SIRT1 and increased activation of RelA/p65 were observed in peripheral lungs of smokers and subjects with COPD. The importance of SIRT1 further gains credence from the observation of McBurney *et al* (91) that genetic ablation of SIRT1 leads to increased neutrophil infiltration in mouse lung, suggesting that knock-down of SIRT1 leads to exaggerated lung inflammation. Hence, it is possible that CS-mediated reduction in SIRT1 may in part be responsible for increased neutrophil influx, NF-κB activation and inflammatory response seen in lungs of smokers and subjects with COPD.

It was further determined the molecular mechanism of SIRT1 reduction and its role in pro-inflammatory cytokine release in response to CS exposure in human macrophage-like cells (MonoMac6) *in vitro.* Alveolar macrophages are considered to be important cells in perpetuating the inflammatory response of CS (60, 74, 92). CSE treatment significantly increased the release of pro-inflammatory cytokine, IL-8, concomitant with decreased levels of SIRT1 protein and mRNA expression in MonoMac6 cells. Recently, Yang *et al.* (75) reported that CS-mediated decrease in SIRT1 levels were correlated with decreased SIRT1 activity in macrophages and rat lungs. Since this decrease was correlated with increased release of IL-8, it was speculated that CSE-mediated decrease in the levels af SIRT1 play an important role in release of pro-inflammatory cytokines. In order to support this observation and to determine the specific effect of SIRT1 in CS-induced pro-inflammatory cytokine release, further experiments were performed on MonoMac6 cells by knocking-down SIRT1 or over-expressing SIRT1 as well as using SIRT1 defective mutant lacking deacetylase activity. Knock-down of endogenous SIRT1 and mutation of SIRT1 deacetylase domain augmented the CS-stimulated pro-inflammatory cytokine (IL-8) release, whereas SIRT1 over-expression resulted in decreased IL-8 release in response to CSE exposure. The present inventors have previously shown that pharmacological activation of SIRT1 reduced the CSE-mediated IL-8 release in MonoMac6 cells (75) and the present findings further support these observations and emphasize the importance of SIRT1 in regulation of pro-inflammatory mediators, such as IL-8 and other NF-κB-dependent genes (matrix metalloproteinases, growth factors and mucin genes). The mechanism whereby CS alters the levels of SIRT1 is not known, but it is possible that SIRT1 is regulated by post-translational modifications and/or by kinase signaling mechanisms. The other possible mechanism would be nucleocytoplasmic shuttling of SIRT1 by kinase signaling mechanism leading to proteasomal degradation of SIRT1 in the cytoplasm.

It is well known that CS-induced oxidative stress is responsible for pro-inflammatory cytokine release in the lung (93). Previously, the present inventors have shown that levels of lipid peroxidation products such as 4-hydroxy-2-nonenal (4-HNE) were increased in lungs of subjects with COPD (94). Post-translational modifications of various proteins by oxidative/nitrosative stress have been shown to have influence on various cellular functions (79, 95, 96). To determine the CS-mediated reduction in SIRT1 levels and its post-translational modifications, SIRT1 modification was evaluated by measuring the SIRT1 adducts with 4-HNE (reactive aldehydes which form protein carbonyls), a highly reactive diffusible product of lipid peroxidation and a key mediator of oxidant-induced cell signaling and apoptosis (97). SIRT1-adducts with 4-HNE and 3-nitrotyrosine in lungs were increased in smokers and subjects with COPD compared to non-smokers. CSE induced the formation of SIRT1-4-HNE adducts in MonoMac6 cells. Cysteine, histidine and lysine, the three nucleophilic amino acids, have been shown to be the target of modification by 4-HNE (98). The high affinity of 4-HNE towards lysine becomes important with respect to SIRT1, because lysine residues 1020/1024 present on the active site domain of SIRT1 may be the direct target of 4-HNE. Thus, the increased SIRT1-4-HNE adducts formation seen after smoke exposure may therefore form a part of the mechanism responsible for the reduction in SIRT1 activity/level. However, it may also be possible that other residues such as cysteine(s) present on SIRT1 is involved in formation of SIRT1-4-HNE adducts. Both oxidation and nitration can damage proteins, nitration of protein tyrosine residues to form 3-nitrotyrosine is considered a hallmark of tissue injury caused by inflammation (95, 96). Post-translationally modified proteins can be a direct target of proteolytic degradation and removal (98). The increased SIRT1 protein tyrosine nitration seen after CSE exposure in MonoMac6 cells may trigger increased proteolytic degradation of this protein, resulting in decreased SIRT1 levels. Thus, the decreased SIRT1 levels in smokers and COPD subjects may be explained on the basis of the CS-mediated oxidative/nitrosative (which occurs in subjects with COPD) alterations on the SIRT1 proteins. In view of the fact that SIRT1 is an anti-aging and antiinflammatory molecule (63, 79), the CS-mediated SIRT1 modification/reduction may have a role in lung inflammation and aging seen in COPD subjects (58, 61). However, it remained to be determined whether SIRT1 reduction is directly associated with the decline in lung function in smokers or disease progession/severity of COPD. Since part of the COPD subjects were ex-smokers and some of them were on inhaled steroids, it is likely that once initiated, the alterations of SIRT1 may not be fully reversible (irreversible epigenetics events), which in turn might be one contributor to the persistence of many inflammatory changes observed even after cessation of smoking.

Reduction/inactivation of other deacetylases, such as HDACs have been reported to cause transactivation of NF-κB and induction of pro-inflammatory cytokine release (79, 85, 88). Recently, Yeung *et al* (73) demonstrated that SIRT1 physically interacts with the RelA/p65 subunit of NF-κB and inhibits gene transcription by deacetylating RelA/p65 at lysine 310, suggesting acetylated lysine 310 might form a platform for the binding of a bromodomain-containing protein that is required for full transcriptional activity of RelA/p65 (99, 100). Since acetylation at lysine 310 is required for full transactivation function of RelA/p65 (86, 87), the levels of acetylated lysine 310 moiety of RelA/p65 subunit of NF-κB in CSE-treated MonoMac6 cells were determined. It was found that CS-mediated SIRT1 reduction was associated with increased acetylation of lysine 310 moiety on RelA/p65. SIRT1 knock down also increased the acetylation of RelA/p65 (K310) acetylation and this effect was further augmented by acetylation effect of CS on RelA/p65. These data indicate that CSE caused acetylation of RelA/p65 NF-κB by reduction of SIRT1 deacetylase level and/or post-translational modifications of its lysine residues. Hence, increased post-translational modifications of SIRT1 lead to disruption of RelA/p65-SIRT1 complex, which would then, culminate into NF-κB acetylation and persistent activation of NF-κB in response to CSE exposure in macrophages. Apart from NF-κB regulation, SIRT1 also regulates stress/protective pathway via deacetylation of the forkhead box class (FOXO3) transcription factor. SIRT1 reduction leads to acetylation of FOXO3 which would then result in loss of its transcription activity for transcription of GADD45 (DNA repair) and MnSOD genes (63). Hence, loss of SIRT1 by CS will lead to acetylation of FOXO3 and tumor suppressor p53 resulting in lung cells senescence and apoptosis.

In conclusion, it was shown for the first time that the level of nuclear SIRT1 protein was decreased in peripheral lung tissue of smokers and subjects with COPD. SIRT1 proteins undergo post-translational covalent modifications in response to CS exposure which may not be fully reversible. These changes render SIRT1 inactive leading to acetylation/activation of RelA/p65 and thereby uncontrolled expression of pro-inflammatory mediators which is seen in macrophages/lungs of smokers and subjects with COPD. In view of the role of SIRT1 in regulation of pro-inflammatory mediators, apoptosis, senescence, cell survival, differentiation and aging, it is tempting to propose that CS-mediated alterations in SIRT1 would have ramifications on these processes which are directly linked to the pathogenesis of COPD (58-60, 62, 78). Further studies are required to understand the mechanism of CS-mediated down-regulation of SIRT1 and its involvement in chronic inflammatory and injurious processes in the lung using genetic gain and loss of function, and whether upregulation or genetic modifications of SIRT1 can attenuate such processes in animal models of COPD.

### References

1. Burchfiel CM, EB Marcus, JD Curb, CJ Maclean, WM Vollmer, LR Johnson, KO Fong, BL Rodriguez, KH Masaki, and AS Buist. Effects of smoking and smoking cessation on longitudinal decline in pulmonaryfunction. Am J Respir Crit Care Med 1995; 151:1778-1785.
2. Yang SR, S Valvo, H Yao, A Kode, S Rajendrasozhan, I Edrisinghe, S Caito, D Adenuga, R Henry, G Fromm, S Maggirwar, JD Li, MD Bulger, and I Rahman. IKK{alpha} Causes Chromatin Modification on Pro-inflammatory Genes by Cigarette Smoke in Mouse Lung. Am J Respir Cell Mol Biol 2008; 38:689-698.
3. Rahman I, and IM Adcock. Oxidative stress and redox regulation of lung inflammation in COPD. EurRespir J 2006; 28:219-242.
4. Yang SR, AS Chida, MR Bauter, N Shafiq, K Seweryniak, SB Maggirwar, I Kilty, and I Rahman. Cigarette smoke induces proinflammatory cytokine release by activation of NF-kappaB and posttranslational modifications of histone deacetylase in macrophages. Am J Physiol Lung Cell Mol Physiol 2006; 291:L46- 57.
5. Szulakowski P, AJ Crowther, LA Jimenez, K Donaldson, R Mayer, TB Leonard, W MacNee, and EM Drost. The effect of smoking on the transcriptional regulation of lung inflammation in patients with chronic obstructive pulmonary disease. Am JRespir Crit Care Med 2006; 174:41-50.
6. Ito K, M Ito, WM Elliott, B Cosio, G Caramori, OM Kon, A Barczyk, S Hayashi, IM Adcock, JC Hogg, and PJ Barnes. Decreased histone deacetylase activity in chronic obstructive pulmonary disease. N Engl J Med 2005; 352:1967-1976.
7. Marwick JA, PA Kirkham, CS Stevenson, H Danahay, J Giddings, K Butler, K Donaldson, W Macnee, and I Rahman. Cigarette smoke alters chromatin remodeling and induces proinflammatory genes in rat lungs. Am JRespir Cell Mol Biol 2004; 31:633-642.
8. Di Stefano A, G Caramori, T Oates, A Capelli, M Lusuardi, I Gnemmi, F Ioli, KF Chung, CF Donner, PJ Barnes, and IM Adcock. Increased expression of nuclear factor-kappaB in bronchial biopsies from smokers and patients with COPD. Eur Respir J 2002; 20:556-563.
9. Morrison D, I Rahman, S Lannan, and W MacNee. Epithelial permeability, inflammation, and oxidant stress in the air spaces of smokers. Am JRespir Crit Care Med 1999; 159:473-479.
10. Keatings VM, PD Collins, DM Scott, and PJ Barnes. Differences in interleukin-8 and tumor necrosis factor-alpha in induced sputum from patients with chronic obstructive pulmonary disease or asthma. Am J Respir Crit Care Med 1996; 153:530-534.
11. Puchelle E, JM Zahm, JM Tournier, and C Coraux. Airway epithelial repair, regeneration, and remodeling after injury in chronic obstructive pulmonary disease. Proc Am Thorac Soc 2006; 3:726-733.
12. Hogg JC, F Chu, S Utokaparch, R Woods, WM Elliott, L Buzatu, RM Cherniack, RM Rogers, FC Sciurba, HO Coxson, and PD Pare. The nature of small-airway obstruction in chronic obstructive pulmonary disease. N Engl J Med 2004; 350:2645-2653.
13. van der Strate BW, DS Postma, CA Brandsma, BN Melgert, MA Luinge, M Geerlings, MN Hylkema, Avan den Berg, W Timens, and HA Kerstjens. Cigarette smoke-induced emphysema: A role for the B cell? Am JRespir Crit Care Med 2006; 173:751-758.
14. Rajendrasozhan S, SR Yang, VL Kinnula, and I Rahman. SIRT1, an antiinflammatory and antiaging protein, is decreased in lungs of patients with chronic obstructive pulmonary disease. Am JRespir Crit Care Med 2008; 177:861-870.
15. Yang SR, J Wright, M Bauter, K Seweryniak, A Kode, and I Rahman. Sirtuin regulates cigarette smoke induced proinflammatory mediator release via RelA/p65 NF-kappaB in macrophages in vitro and in rat lungs in vivo: implications for chronic inflammation and aging. Am J Physiol Lung Cell Mol Physiol 2007; 292:L567-576.
16. Rahman I. Oxidative stress, transcription factors and chromatin remodelling in lung inflammation. Biochem Pharmacol 2002; 64:935-942.
17. Barnes PJ, and M Karin. Nuclear factor-kappaB: a pivotal transcription factor in chronic inflammatory diseases. N Engl J Med 1997; 336:1066-1071.
18. Murphy TF. The role of bacteria in airway inflammation in exacerbations of chronic obstructive pulmonary disease. Curr Opin Infect Dis 2006; 19:225-230.
19. Yagi O, K Aoshiba, and A Nagai. Activation of nuclear factor-kappaB in airway epithelial cells in patients with chronic obstructive pulmonary disease. Respiration 2006; 73:610-616.
20. Xia Y, S Chen, Y Wang, N Mackman, G Ku, D Lo, and L Feng. RelB modulation of Ikappa B alpha stability as a mechanism of transcription suppression of interleukin 1alpha (IL-1alpha), IL-1beta, and tumor necrosis factor alpha in fibroblasts. Mol Cell Biol 1999; 19:7688-7696.
21. Xia Y, ME Pauza, L Feng, and D Lo. RelB regulation of chemokine expression modulates local inflammation. Am J Pathol 1997; 151:375-387.
22. Mills DM, G Bonizzi, M Karin, and RC Rickert. Regulation of late B cell differentiation by intrinsic IKKalpha-dependent signals. Proc Natl Acad Sci USA 2007; 104:6359-6364.
23. Beinke S, and SC Ley. Functions of NF-kappaB1 and NF-kappaB2 in immune cell biology. Biochem J2004; 382:393-409.
24. Marienfeld R, F Berberich-Siebelt, I Berberich, A Denk, E Serfling, and M Neumann. Signal-specific and phosphorylation-dependent RelB degradation: a potential mechanism of NF-kappaB control. Oncogene 2001; 20:8142-8147.
25. Marienfeld R, MJ May, I Berberich, E Serfling, S Ghosh, and M Neumann. RelB forms transcriptionally inactive complexes with ReIA/p65. J Biol Chem 2003; 278:19852-19860.
26. Solan NJ, H Miyoshi, EM Carmona, GD Bren, and CV Paya. RelB cellular regulation and transcriptional activity are regulated by p100. J Biol Chem 2002; 277:1405-1418.
27. Bonizzi G, M Bebien, DC Otero, KE Johnson-Vroom, Y Cao, D Vu, AG Jegga, BJ Aronow, G Ghosh, RC Rickert, and M Karin. Activation of IKKalpha target genes depends on recognition of specific kappaB binding sites by RelB:p52 dimers. Embo J 2004; 23:4202-4210.
28. Fusco AJ, OV Savinova, R Talwar, JD Kearns, A Hoffmann, and G Ghosh. Stabilization of RelB Requires Multidomain Interactions with p100/p52. J Biol Chem 2008; 283:12324-12332.
29. Foronjy RF, O Mirochnitchenko, O Propokenko, V Lemaitre, Y Jia, M Inouye, Y Okada, and JM D'Armiento. Superoxide dismutase expression attenuates cigarette smoke- or elastase-generated emphysema in mice. Am JRespir Crit Care Med 2006; 173:623-631.
30. Thatcher TH, NA McHugh, RW Egan, RW Chapman, JA Hey, CK Turner, MR Redonnet, KE Seweryniak, PJ Sime, and RP Phipps. Role of CXCR2 in cigarette smoke-induced lung inflammation. Am JPhysiol Lung Cell Mol Physiol 2005; 289:L322-328.
31. Thatcher TH, SB Maggirwar, CJ Baglole, HF Lakatos, TA Gasiewicz, RP Phipps, and PJ Sime. Aryl hydrocarbon receptor-deficient mice develop heightened inflammatory responses to cigarette smoke and endotoxin associated with rapid loss of the nuclear factor-kappaB component RelB. Am J Pathol 2007; 170:855-864.
32. Yao H, I Edirisinghe, SR Yang, S Rajendrasozhan, A Kode, S Caito, D Adenuga, and I Rahman. Genetic ablation of NADPH oxidase enhances susceptibility to cigarette smoke-induced lung inflammation and emphysema in mice. Am JPathol 2008; 172:1222-1237.
33. Yao H, I Edirisinghe, S Rajendrasozhan, SR Yang, S Caito, D Adenuga, and I Rahman. Cigarette smokemediated inflammatory and oxidative responses are strain dependent in mice. Am JPhysiol Lung Cell Mol Physiol 2008.
34. Zhou LJ, DC Ord, SA Omori, and TF Tedder. Structure of the genes encoding the CD19 antigen of human and mouse B lymphocytes. Immunogenetics 1992; 35:102-111.
35. Moesby L, S Jensen, EW Hansen, and JD Christensen. A comparative study of Mono Mac 6 cells, isolated mononuclear cells and Limulus amoebocyte lysate assay in pyrogen testing. Int J Pharm 1999; 191:141-149.
36. Ziegler-Heitbrock HW, E Thiel, A Futterer, V Herzog, A Wirtz, and G Riethmuller. Establishment of a human cell line (Mono Mac 6) with characteristics of mature monocytes. Int J Cancer 1988; 41:456-461.
37. Klein G, B Giovanella, A Westman, JS Stehlin, and D Mumford. An EBV-genome-negative cell line established from an American Burkitt lymphoma; receptor characteristics. EBV infectibility and permanent conversion into EBV-positive sublines by in vitro infection. Intervirology 1975; 5:319-334.
38. McCabe MJ, Jr., MD Laiosa, L Li, SL Menard, RR Mattingly, and AJ Rosenspire. Low and nontoxic inorganic mercury burdens attenuate BCR-mediated signal transduction. Toxicol Sci 2007; 99:512-521.
39. Carp H, and A Janoff. Possible mechanisms of emphysema in smokers. In vitro suppression of serum elastase-inhibitory capacity by fresh cigarette smoke and its prevention by antioxidants. Am Rev Respir Dis 1978; 118:617-621.
40. Moodie FM, JA Marwick, CS Anderson, P Szulakowski, SK Biswas, MR Bauter, I Kilty, and I Rahman. Oxidative stress and cigarette smoke alter chromatin remodeling but differentially regulate NF-kappaB activation and proinflammatory cytokine release in alveolar epithelial cells. Faseb J 2004; 18:1897-1899.
41. Kode A, SR Yang, and I Rahman. Differential effects of cigarette smoke on oxidative stress and proinflammatory cytokine release in primary human airway epithelial cells and in a variety of transformed alveolar epithelial cells. Respir Res 2006; 7:132-151.
42. Kweon SM, B Wang, D Rixter, JH Lim, T Koga, H Ishinaga, LF Chen, H Jono, H Xu, and JD Li. Synergistic activation of NF-kappaB by nontypeable H. influenzae and S. pneumoniae is mediated by CK2, IKKbeta-IkappaBalpha, and p38 MAPK. Biochem Biophys Res Commun 2006; 351:368-375.
43. Bulger M, D Schubeler, MA Bender, J Hamilton, CM Farrell, RC Hardison, and M Groudine. A complex chromatin landscape revealed by patterns of nuclease sensitivity and histone modification within the mouse beta-globin locus. Mol Cell Biol 2003; 23:5234-5244.
44. Dejardin E. The alternative NF-kappaB pathway from biochemistry to biology: pitfalls and promises for future drug development. Biochem Pharmacol 2006; 72:1161-1179.
45. Xiao G, AB Rabson, W Young, G Qing, and Z Qu. Alternative pathways of NF-kappaB activation: a double-edged sword in health and disease. Cytokine Growth Factor Rev 2006; 17:281-293.
46. Yoza BK, JY Hu, SL Cousart, LM Forrest, and CE McCall. Induction of RelB participates in endotoxin tolerance. J Immunol 2006; 177:4080-4085.
47. Lernbecher T, B Kistler, and T Wirth. Two distinct mechanisms contribute to the constitutive activation of RelB in lymphoid cells. Embo J 1994; 13:4060-4069.
48. Yilmaz ZB, DS Weih, V Sivakumar, and F Weih. RelB is required for Peyer's patch development: differential regulation of p52-RelB by lymphotoxin and TNF. Embo J 2003; 22:121-130.
49. Bren GD, NJ Solan, H Miyoshi, KN Pennington, LJ Pobst, and CV Paya. Transcription of the RelB gene is regulated by NF-kappaB. Oncogene 2001; 20:7722-7733.
50. Saccani S, S Pantano, and G Natoli. Modulation of NF-kappaB activity by exchange of dimers. Mol Cell 2003; 11: 1563-1574.
51. Vogel CF, E Sciullo, and F Matsumura. Involvement of RelB in aryl hydrocarbon receptor-mediated induction of chemokines. Biochem Biophys Res Commun 2007; 363:722-726.
52. Schneider G, D Saur, JT Siveke, R Fritsch, FR Greten, and RM Schmid. IKKalpha controls p52/RelB at the skp2 gene promoter to regulate G1- to S-phase progression. Embo J2006; 25:3801-3812.
53. Park GY, X Wang, N Hu, TV Pedchenko, TS Blackwell, and JW Christman. NIK is involved in nucleosomal regulation by enhancing histone H3 phosphorylation by IKKalpha. J Biol Chem 2006; 281:18684-18690.
54. Weih F, D Carrasco, SK Durham, DS Barton, CA Rizzo, RP Ryseck, SA Lira, and R Bravo. Multiorgan inflammation and hematopoietic abnormalities in mice with a targeted disruption of RelB, a member of the NF-kappa B/Rel family. Cell 1995; 80:331-340.
55. Pauwels RA, Buist AS, Calverley PM, Jenkins CR, Hurd SS. Global strategy for the diagnosis, management, and prevention of chronic obstructive pulmonary disease. NHLBI/WHO Global Initiative for Chronic Obstructive Lung Disease (GOLD) Workshop summary. Am J Respir Crit Care Med 2001;163:1256-1276.
56. Murray CJ, Lopez AD. Mortality by cause for eight regions of the world: Global Burden of Disease Study. Lancet 1997;349:1269-1276.
57. Vogelmeier C, Bals R. Chronic obstructive pulmonary disease and premature aging. Am JRespir Crit Care Med 2007; 175:1217-1218.
58. Tuder RM. Aging and cigarette smoke: fueling the fire. Am JRespir Crit Care Med 2006;174:490-491.
59. Barnes PJ. Chronic obstructive pulmonary disease. NEngl J Med 2000;343:269-280.
60. Barnes PJ, Shapiro SD, Pauwels RA. Chronic obstructive pulmonary disease: molecular and cellular mechanisms. Eur Respir J 2003;22:672-688.
61. Burchfiel CM, Marcus EB, Curb JD, Maclean CJ, Vollmer WM, Johnson LR, Fong KO, Rodriguez BL, Masaki KH, Buist AS. Effects of smoking and smoking cessation on longitudinal decline in pulmonary function. Am J Respir Crit Care Med 1995;151:1778-1785.
62. Hogg JC, Chu F, Utokaparch S, Woods R, Elliott WM, Buzatu L, Cherniack RM, Rogers RM, Sciurba FC, Coxson HO, Pare PD. The nature of small-airway obstruction in chronic obstructive pulmonary disease. N Engl J Med 2004;350:2645-2653.
63. Michan S, Sinclair D. Sirtuins in mammals: insights into their biological function. Biochem J2007;404:1-13*.*
64. Lin SJ, Defossez PA, Guarente L. Requirement of NAD and SIR2 for life-span extension by calorie restriction in Saccharomyces cerevisiae. Science 2000;289:2126-2128.
65. Tissenbaum HA, Guarente L. Increased dosage of a sir-2 gene extends lifespan in Caenorhabditis elegans. Nature 2001;410:227-230.
66. Bitterman KJ, Anderson RM, Cohen HY, Latorre-Esteves M, Sinclair DA. Inhibition of silencing and accelerated aging by nicotinamide, a putative negative regulator of yeast sir2 and human SIRT1. J Biol Chem 2002;277:45099-45107.
67. Anderson RM, Bitterman KJ, Wood JG, Medvedik O, Sinclair DA. Nicotinamide and PNC1 govern lifespan extension by calorie restriction in Saccharomyces cerevisiae. Nature 2003;423:181-185.
68. Howitz KT, Bitterman KJ, Cohen HY, Lamming DW, Lavu S, Wood JG, Zipkin RE, Chung P, Kasielewski A, Zhang LL, Scherer B, Sinclair DA. Small molecule activators of sirtuins extend Saccharomyces cerevisiae lifespan. Nature 2003;425:191-196.
69. Trapp J, Jung M. The role ofNAD+ dependent histone deacetylases (sirtuins) in ageing. Curr Drug Targets 2006;7:1553-1560.
70. Sauve AA, Schramm VL. SIR2: the biochemical mechanism of NAD(+)-dependent protein deacetylation and ADP-ribosyl enzyme intermediates. Curr Med Chem 2004;11:807-826.
71. Grubisha O, Smith BC, Denu JM. Small molecule regulation of Sir2 protein deacetylases. FEBS J 2005;272:4607-4616.
72. Sauve AA, Wolberger C, Schramm VL, Boeke JD. The biochemistry of sirtuins. Annu Rev Biochem 2006;75:435-465.
73. Yeung F, Hoberg JE, Ramsey CS, Keller MD, Jones DR, Frye RA, Mayo MW. Modulation of NF-kappaB-dependent transcription and cell survival by the SIRT1 deacetylase. EMBO J 2004;23:2369-2380.
74. Guarente L, Picard F. Calorie restriction--the SIR2 connection. Cell 2005;120:473-482.
75. Yang SR, Wright J, Bauter M, Seweryniak K, Kode A, Rahman I. Sirtuin regulates cigarette smoke-induced proinflammatory mediator release via RelA/p65 NF-kappaB in macrophages in vitro and in rat lungs in vivo: implications for chronic inflammation and aging. Am J Physiol Lung Cell Mol Physiol 2007;292:L567-576.
76. Jeffery PK. Comparison of the structural and inflammatory features of COPD and asthma. Giles F. Filley Lecture. Chest 2000;117:251S-260S.
77. Saetta M, Turato G, Baraldo S, Zanin A, Braccioni F, Mapp CE, Maestrelli P, Cavallesco G, Papi A, Fabbri LM. Goblet cell hyperplasia and epithelial inflammation in peripheral airways of smokers with both symptoms of chronic bronchitis and chronic airflow limitation. Am J Respir Crit Care Med 2000;161:1016-1021.
78. Shapiro SD. The macrophage in chronic obstructive pulmonary disease. Am J Respir Crit Care Med 1999;160:S29-32.
79. Marwick JA, Kirkham PA, Stevenson CS, Danahay H, Giddings J, Butler K, Donaldson K, Macnee W, Rahman I. Cigarette smoke alters chromatin remodeling and induces proinflammatory genes in rat lungs. Am JRespir Cell Mol Biol 2004;31:633-642.
80. Ito K, Ito M, Elliott WM, Cosio B, Caramori G, Kon OM, Barczyk A, Hayashi S, Adcock IM, Hogg JC, Barnes PJ. Decreased histone deacetylase activity in chronic obstructive pulmonary disease. N Engl J Med 2005;352:1967-1976.
81. Dail DH, Liebow AA, Gmelich JT, Friedman PJ, Miyai K, Myer W, Patterson SD, Hammar SP. Intravascular, bronchiolar, and alveolar tumor of the lung (IVBAT). An analysis of twenty cases of a peculiar sclerosing endothelial tumor. Cancer 1983;51:452-464.
82. Moesby L, Jensen S, Hansen EW, Christensen JD. A comparative study of Mono Mac 6 cells, isolated mononuclear cells and Limulus amoebocyte lysate assay in pyrogen testing. Int J Pharm 1999;191:141-149.
83. Ziegler-Heitbrock HW, Thiel E, Futterer A, Herzog V, Wirtz A, Riethmuller G. Establishment of a human cell line (Mono Mac 6) with characteristics of mature monocytes. Int J Cancer 1988;41:456-461.
84. Carp H, Janoff A. Possible mechanisms of emphysema in smokers. In vitro suppression of serum elastase-inhibitory capacity by fresh cigarette smoke and its prevention by antioxidants. Am Rev Respir Dis 1978;118:617-621.
85. Moodie FM, Marwick JA, Anderson CS, Szulakowski P, Biswas SK, Bauter MR, Kilty I, Rahman I. Oxidative stress and cigarette smoke alter chromatin remodeling but differentially regulate NF-kappaB activation and proinflammatory cytokine release in alveolar epithelial cells. Faseb J 2004;18:1897-1899.
86. Chen LF, Mu Y, Greene WC. Acetylation of RelA at discrete sites regulates distinct nuclear functions ofNF-kappaB. EMBO J 2002;21:6539-6548.
87. Chen LF, Williams SA, Mu Y, Nakano H, Duerr JM, Buckbinder L, Greene WC. NF-kappaB RelA phosphorylation regulates RelA acetylation. Mol Cell Biol 2005;25:7966-7975.
88. Yang SR, Chida AS, Bauter MR, Shafiq N, Seweryniak K, Maggirwar SB, Kilty I, Rahman I. Cigarette smoke induces proinflammatory cytokine release by activation of NF-kappaB and posttranslational modifications of histone deacetylase in macrophages. Am J Physiol Lung Cell Mol Physiol 2006;291:L46-57.
89. Vaquero A, Scher M, Lee D, Erdjument-Bromage H, Tempst P, Reinberg D. Human SirT1 interacts with histone H1 and promotes formation of facultative heterochromatin. Mol Cell 2004;16:93-105.
90. Clayton AL, Hazzalin CA, Mahadevan LC. Enhanced histone acetylation and transcription: a dynamic perspective. Mol Cell 2006;23:289-296.
91. McBurney MW, Yang X, Jardine K, Hixon M, Boekelheide K, Webb JR, Lansdorp PM, Lemieux M. The mammalian SIR2alpha protein has a role in embryagenesis and gametogenesis. Mol Cell Biol 2003;23:38-54.
92. Barnes PJ. Alveolar macrophages in chronic obstructive pulmonary disease (COPD). Cell Mol Biol (Noisy-le-grand) 2004;50 Online Pub:OL627-637.
93. Rahman I, Adcock IM. Oxidative stress and redox regulation of lung inflammation in COPD. Eur Respir J 2006;28:219-242.
94. Rahman I, van Schadewijk AA, Crowther AJ, Hiemstra PS, Stolk J, MacNee W, De Boer WI. 4-Hydroxy-2-nonenal, a specific lipid peroxidation product, is elevated in lungs of patients with chronic obstructive pulmonary disease. Am J Respir Crit Care Med 2002;166:490-495.
95. Cecarini V, Gee J, Fioretti E, Amici M, Angeletti M, Eleuteri AM, Keller JN. Protein oxidation and cellular homeostasis: Emphasis on metabolism. Biochim Biophys Acta 2007;1773:93-104.
96. Ischiropoulos H. Biological tyrosine nitration: a pathophysiological function of nitric oxide and reactive oxygen species. Arch Biochem Biophys 1998;356:1-11.
97. Esterbauer H, Schaur RJ, Zollner H. Chemistry and biochemistry of 4-hydroxynonenal, malonaldehyde and related aldehydes. Free Radic Biol Med 1991;11:81-128.
98. Souza JM, Choi I, Chen Q, Weisse M, Daikhin E, Yudkoff M, Obin M, Ara J, Horwitz J, Ischiropoulos H. Proteolytic degradation of tyrosine nitrated proteins. Arch Biochem Biophys 2000;380:360-366.
99. Dhalluin C, Carlson JE, Zeng L, He C, Aggarwal AK, Zhou MM. Structure and ligand of a histone acetyltransferase bromodomain. Nature 1999;399:491-496.
100. Polesskaya A, Naguibneva I, Duquet A, Bengal E, Robin P, Harel-Bellan A. Interaction between acetylated MyoD and the bromodomain of CBP and/or p300. Mol Cell Biol 2001;21:5312-5320.
101. Moodie FM, Marwick JA, Anderson CS, et al. Oxidative stress and cigarette smoke alter chromatin remodeling but differentially regulate NF-kappaB activation and proinflammatory cytokine release in alveolar epithelial cells. FASEB J 2004;18:1897-1899.
102. Yang SR, Wright J, Bauter M, Seweryniak K, Kode A, Rahman I. Sirtuin regulates cigarette smoke-induced proinflammatory mediator release via RelA/p65 NF-kappaB in macrophages in vitro and in rat lungs in vivo: implications for chronic inflammation and aging. Am JPhysiol Lung Cell Mol Physiol 2007;292:L567-L576.
103. Carp H, Janoff A. Possible mechanisms of emphysema in smokers. In vitro suppression of serum elastase-inhibitory capacity by fresh cigarette smoke and its prevention by antioxidants. Am Rev Respir Dis 1978;118:617-621.
104. Toyokuni S. Reactive oxygen species-induced molecular damage and its application in pathology. Pathol Int 1999;49:91-102.

### Embodiments

Embodiment 1. A method for the treatment of an inflammatory disorder in a subject comprising administering to the subject a medicament comprising:
   a compound that increases the cellular activity of RelB; and
   one or more pharmaceutically acceptable excipients,
Embodiment 2. The method of embodiment 1, wherein the compound is a small molecule or pharmaceutically acceptable salt thereof.
Embodiment 3. The method of embodiment 1, wherein the compound is a biological macromolecule.
Embodiment 4. The method of embodiment 1, wherein the compound increases the formation of RelB -RelA heterodimers.
Embodiment 5. The method of embodiment 1, wherein the inflammatory disorder is chronic obstructive pulmonary disorder.
Embodiment 6. The method of embodiment 1, wherein the inflammatory disorder is endothelial and skeletal muscle disfunction associated with smoking.
Embodiment 7. The method of embodiment 1, wherein the inflammatory disorder is rheumatoid arthritis.
Embodiment 8. The method of embodiment 1, wherein the inflammatory disorder is asthma.
Embodiment 9. The method of embodiment 1, wherein the inflammatory disorder is idiopathic pulmonary fibrosis.
Embodiment 10. A method for the treatment of an inflammatory disorder in a subject comprising administering to the subject a nucleic acid comprising a nucleic acid sequence encoding an amino acid having at least about 90% sequence similarity to SEQ ID NO. 1.
Embodiment 11. The method of embodiment 10, wherein the amino acid sequence has at least about 95% sequence similarity to SEQ ID NO. 1.
Embodiment 12. The method of embodiment 11, wherein the amino acid sequence has at least about 98% sequence similarity to SEQ ID NO. 1.
Embodiment 13. The method of embodiment 10, wherein the amino acid sequence which binds more tightly to RelA than the amino acid encoded by SEQ ID NO. 1.
Embodiment 14. The method of embodiment 10, wherein the nucleic acid sequence encodes an amino acid that is resistant to proteolysis.
Embodiment 15. The method of embodiment 10, wherein the inflammatory disorder is chronic obstructive pulmonary disorder.
Embodiment 16. The method of embodiment 10, wherein the inflammatory disorder is endothelial and skeletal muscle disfunction associated with smoking.
Embodiment 17. The method of embodiment 10, wherein the inflammatory disorder is rheumatoid arthritis.
Embodiment 18. The method of embodiment 10, wherein the inflammatory disorder is asthma.
Embodiment 19. The method of embodiment 10, wherein the inflammatory disorder is idiopathic pulmonary fibrosis.
Embodiment 20. A method for the treatment of an inflammatory disorder in a subject comprising causing one or more cells in the subject to produce endogenous RelB at a concentration higher than the endogenous RelB concentration in an unmodified cell.
Embodiment 21. The method of embodiment 20, wherein said one or more cells are caused to produce RelB by modifying the promoter of the endogenous *RelB* gene.
Embodiment 22. The method of embodiment 20, wherein said one or more cells are caused to produce RelB by administering to the cell a factor that causes increased RelB transcription.
Embodiment 23. A method for the treatment of an inflammatory disorder in a subject comprising administering to the subject a medicament comprising:
   a compound that increases the cellular activity of SIRTI ; and
   one or more pharmaceutically acceptable excipients.
Embodiment 24. The method of embodiment 23, wherein the compound is a small molecule or pharmaceutically acceptable salt thereof.
Embodiment 25. The method of embodiment 23, wherein the compound is a biological macromolecule.
Embodiment 26. The method of embodiment 23, wherein the compound increases the histone deacetylase activity of SIRTI.
Embodiment 27. The method of embodiment 23, wherein the inflammatory disorder is chronic obstructive pulmonary disorder.
Embodiment 28. The method of embodiment 23, wherein the inflammatory disorder is endothelial and skeletal muscle disfunction associated with smoking.
Embodiment 29. The method of embodiment 23, wherein the inflammatory disorder is rheumatoid arthritis.
Embodiment 30. The method of embodiment 23, wherein the inflammatory disorder is asthma.
Embodiment 31. The method of embodiment 23, wherein the inflammatory disorder is idiopathic pulmonary fibrosis.
Embodiment 32. A method for the treatment of an inflammatory disorder in a subject comprising administering to the subject a nucleic acid comprising a nucleic acid sequence encoding an amino acid having at least about 90% sequence similarity to SEQ ID NO. 2.
Embodiment 33. The method of embodiment 32, wherein the amino acid sequence has at least about 95% sequence similarity to SEQ ID NO. 2.
Embodiment 34. The method of embodiment 33, wherein the amino acid sequence has at least about 98% sequence similarity to SEQ ID NO. 2.
Embodiment 35. The method of embodiment 32, wherein the amino acid sequence which binds more tightly to RelA than the amino acid encoded by SEQ ID NO. 2.
Embodiment 36. The method of embodiment 32, wherein the nucleic acid sequence encodes an amino acid that is resistant to proteolysis.
Embodiment 37. The method of embodiment 32, wherein the inflammatory disorder is chronic obstructive pulmonary disorder.
Embodiment 38. The method of embodiment 32, wherein the inflammatory disorder is endothelial and skeletal muscle disfunction associated with smoking.
Embodiment 39. The method of embodiment 32, wherein the inflammatory disorder is rheumatoid arthritis.
Embodiment 40. The method of embodiment 32, wherein the inflammatory disorder is asthma.
Embodiment 41. The method of embodiment 32, wherein the inflammatory disorder is idiopathic pulmonary fibrosis.
Embodiment 42. A method for the treatment of an inflammatory disorder in a subject comprising causing one or more cells in the subject to produce endogenous SIRTI at a concentration higher than the endogenous SIRTI concentration in an unmodified cell.
Embodiment 43. The method of embodiment 42, wherein said one or more cells are caused to produce SIRTI by modifying the promoter of the endogenous *Sirtl gene.*
Embodiment 44. The method of embodiment 42, wherein said one or more cells are caused to produce SIRTI by administering to the cell a factor that causes increased SIRTI transcription.

## Claims

1. A compound that increases the cellular activity of SIRT1, and one or more pharmaceutically acceptable excipients, for use in the treatment of an inflammatory disorder.

2. A nucleic acid sequence encoding an amino acid having at least about 90% sequence similarity to SEQ ID NO. 2, for use in the treatment of an inflammatory disorder.

3. A molecule that causes one or more cells in a subject to produce endogenous SIRT1 at a concentration higher than the endogenous SIRT1 concentration in an unmodified cell, for use in the treatment of an inflammatory disorder.

4. The use of claim 1, wherein the compound is a small molecule or pharmaceutically acceptable salt thereof; or a biological macromolecule.

5. The use of any of claims 1 to 4, wherein the inflammatory disorder is:
(i) chronic obstructive pulmonary disorder; or
(ii) endothelial and skeletal muscle disfunction associated with smoking; or
(iii) rheumatoid arthritis; or
(iv) asthma; or
(v) idiopathic pulmonary fibrosis.

6. The use of claim 2, wherein the nucleic acid sequence encodes an amino acid that is resistant to proteolysis.

7. The use of claim 1, wherein the compound increases the histone deacetylase activity of SIRT1.

8. The use of claim 2, wherein the amino acid sequence has at least about 95% sequence similarity to SEQ ID NO. 2, preferably at least 98% sequence similarity to SEQ ID NO. 2.

9. The use of claim 2, wherein the amino acid sequence binds more tightly to RelA than the amino acid encoded by SEQ ID NO. 2.

10. The use of claim 3, wherein the molecule is a promoter of the endogenous *Sirt1* gene; or is a factor that causes increased SIRT1 transcription.
